# EUROPEAN PATENT APPLICATION

(11) **EP 4 293 105 A1**
(43) Date of publication of application: **20.12.2023**
(21) Application number: 22752782.7
(22) Date of filing: 09.02.2022
(51) Int. Cl.: C12N 5/02, C12N 5/071, C12N 5/10

(54) **MATURATION AGENT**

(30) Priority: 09.02.2021 JP 2021019120
(71) Applicant: Orizuru Therapeutics, Inc., Fujisawa-shi Kanagawa 251-8555 (JP)
(72) Inventor: HIYOSHI Hideyuki, Fujisawa-shi, Kanagawa 251-0012 (JP); YAMAZOE Noriko, Fujisawa-shi, Kanagawa 251-0012 (JP); TOYODA Taro, Kyoto-shi, Kyoto 606-8501 (JP); KONAGAYA Shuhei, Kyoto-shi, Kyoto 606-8501 (JP)
(74) Representative: Jones, Nicholas Andrew
(86) International application number: PCT/JP2022/005137
(87) International publication number: WO 2022/172960

(57) **Abstract**

An object of the present invention is to provide a novel method for inducing the differentiation of pluripotent stem cells into an insulin-producing cell population. The present invention provides a method for producing an insulin-producing cell population, comprising culturing a pancreatic endocrine progenitor cell population and/or a cell population at a later stage of differentiation to be treated in a medium containing a compound having tubulin polymerization-promoting activity and/or tubulin depolymerization-inhibiting activity.

## Description

### Technical Field

The present invention relates to a method for producing an insulin-producing cell population from pluripotent stem cells. More specifically, the present invention relates to a method for producing an insulin-producing cell population, comprising treating a pancreatic endocrine progenitor cell population or a cell population at a later stage of differentiation, obtained by the induction of differentiation from pluripotent stem cells with a compound having tubulin polymerization-promoting activity and/or tubulin depolymerization-inhibiting activity.

### [Background Art]

Research is underway to induce the differentiation of pluripotent stem cells such as iPS cells or ES cells into insulin-producing cells or pancreatic β cells and to apply the obtained cells to the treatment of diabetes mellitus.

Various approaches have been developed and reported so far in order to induce the differentiation of pluripotent stem cells into insulin-producing cell populations (Non Patent Literature 1). However, an insulin-producing cell population obtained by the induction of differentiation comprises various cells in addition to the cells of interest such as insulin-producing cells. In the case of applying an insulin-producing cell population to the treatment of diabetes mellitus, it is very important from a safety standpoint to strictly control the types of cells contained in the cell population. Hence, there has been a strong demand for a novel method for inducing differentiation into an insulin-producing cell population capable of reducing coexisting unintended cells.

### Citation List

### Non Patent Literature

Non Patent Literature 1: Stem Cell Research (2015) 14, 185-197

### Summary of Invention

### Technical Problem

An object of the present invention is to provide a novel method for inducing the differentiation of pluripotent stem cells into an insulin-producing cell population.

### Solution to Problem

The present inventors have conducted diligent studies to attain the object and consequently found that an insulin-producing cell population obtained by the induction of differentiation from pluripotent stem cells is subjected to extended culture using a basal medium, whereby even unintended cells (proliferative non-endocrine cells) slightly remaining in the cell population can be detected with high sensitivity.

The present inventors have also found that in the process of inducing an insulin-producing cell population from pluripotent stem cells, a pancreatic endocrine progenitor cell population and/or a cell population at a later stage of differentiation is cultured in a medium containing a compound having tubulin polymerization-promoting activity and/or tubulin depolymerization-inhibiting activity (hereinafter, also referred to as a "microtubule inhibitor"), whereby the unintended cells present in the resulting insulin-producing cell population can be reduced.

The present invention is based on these novel findings and encompasses the following inventions.
[1] A method for producing an insulin-producing cell population, comprising
   culturing a pancreatic endocrine progenitor cell population and/or a cell population at a later stage of differentiation to be treated in a medium containing a compound having tubulin polymerization-promoting activity and/or tubulin depolymerization-inhibiting activity.
[2] The method according to [1], wherein the compound having tubulin polymerization-promoting activity and/or tubulin depolymerization-inhibiting activity is a compound selected from taxoid anticancer agents or a pharmacologically acceptable salt thereof.
[3] The method according to [1] or [2], wherein the compound having tubulin polymerization-promoting activity and/or tubulin depolymerization-inhibiting activity is docetaxel or a pharmacologically acceptable salt thereof.
[4] The method according to any of [1] to [3], wherein the produced insulin-producing cell population comprises 95% or more of CHGA-positive cells.
[5] The method according to any of [1] to [3], wherein the produced insulin-producing cell population comprises 0.1% or less of CHGA-negative and Ki67-positive cells.
[6] The method according to any of [1] to [5], wherein the pancreatic endocrine progenitor cell population and/or the cell population at a later stage of differentiation to be treated is a cell population produced by the induction of differentiation from pluripotent stem cells.
[7] A culture medium for a pancreatic endocrine progenitor cell population and/or a cell population at a later stage of differentiation, comprising a compound having tubulin polymerization-promoting activity and/or tubulin depolymerization-inhibiting activity.
[8] The culture medium according to [7], wherein the compound having tubulin polymerization-promoting activity and/or tubulin depolymerization-inhibiting activity is a compound selected from taxoid anticancer agents or a pharmacologically acceptable salt thereof.
[9] The culture medium according to [7] or [8], wherein the compound having tubulin polymerization-promoting activity and/or tubulin depolymerization-inhibiting activity is docetaxel or a pharmacologically acceptable salt thereof.
[10] The culture medium according to any of [7] to [9], wherein the culture medium is used for increasing the proportion of CHGA-positive cells and/or for increasing the proportion of insulin-positive and NKX6.1-positive cells.
[10-1] A method for detecting non-endocrine cells in an insulin-producing cell population, comprising culturing the insulin-producing cell population in a medium containing a growth factor.
[10-2] A method for detecting non-endocrine cells in an insulin-producing cell population, comprising culturing the insulin-producing cell population in a medium containing epidermal growth factor (EGF) or a substance having equivalent or similar activity thereto.
[11] A method for detecting non-endocrine cells in an insulin-producing cell population, comprising culturing the insulin-producing cell population in a medium containing epidermal growth factor (EGF).
[12] The method according to [11], wherein the non-endocrine cells are CHGA-negative and PDX1-positive cells and/or CHGA-negative and PDX1-negative cells.
[13] A method for removing non-endocrine cells, comprising
   culturing a pancreatic endocrine progenitor cell population and/or a cell population at a later stage of differentiation to be treated in a medium containing a compound having tubulin polymerization-promoting activity and/or tubulin depolymerization-inhibiting activity.
[13-1] A culture medium for an insulin-producing cell population, comprising a growth factor, wherein the culture medium is used for detecting non-endocrine cells in the insulin-producing cell population.
[13-2] A culture medium for an insulin-producing cell population, comprising epidermal growth factor (EGF) or a substance having equivalent or similar activity thereto, wherein the culture medium is used for detecting non-endocrine cells in the insulin-producing cell population.
[14] A culture medium for an insulin-producing cell population, comprising EGF, wherein the culture medium is used for detecting non-endocrine cells in the insulin-producing cell population.

The present specification encompasses the contents described in the specification, etc. of Japanese Patent Application No. 2021-019120 filed on February 9, 2021 on which the priority of the present application is based.

All publications, patents and patent applications cited herein are incorporated herein by reference in their entirety.

### Advantageous Effects of Invention

The present invention can provide a novel method for inducing the differentiation of pluripotent stem cells into an insulin-producing cell population. The present invention can produce an insulin-producing cell population in which coexisting unintended cells have been reduced.

### Brief Description of Drawings

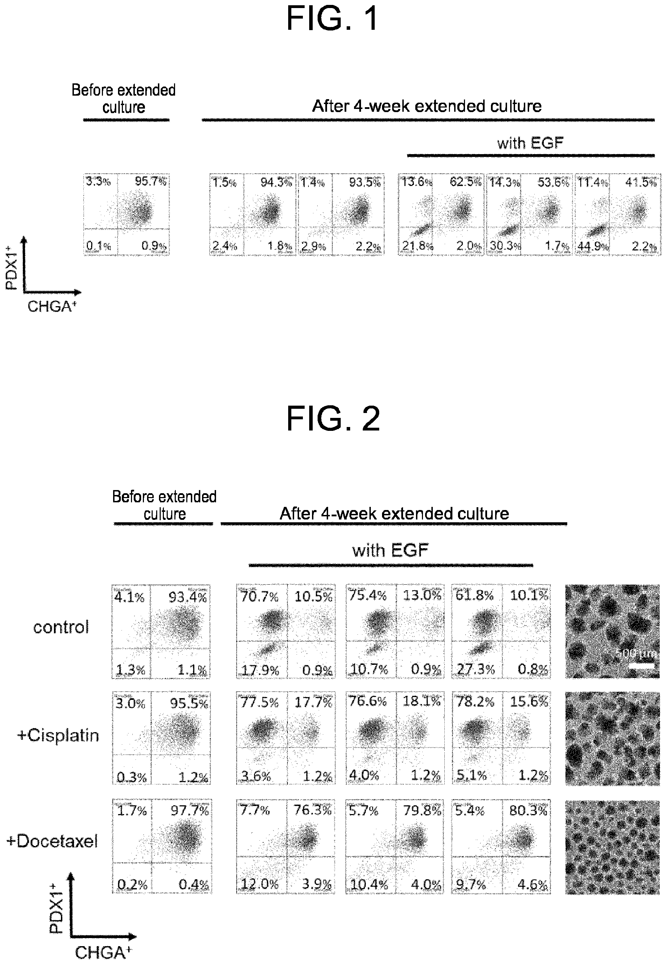

[Figure 1] Figure 1 shows results of analyzing the expression of marker proteins (PDX1 and CHGA) by flow cytometry in an insulin-producing cell population extended-cultured in a basal medium containing epidermal growth factor (EGF).
[Figure 2] Figure 2 shows results of extended-culturing an insulin-producing cell population obtained by treatment with cisplatin or docetaxel in a basal medium containing EGF, and then analyzing the expression of marker proteins (PDX1 and CHGA) in each resulting cell population by flow cytometry, and a photograph of each cell population thus extended-cultured. The control cell population is an insulin-producing cell population obtained without the use of any of cisplatin and docetaxel, and was extended-cultured in the same manner as above.

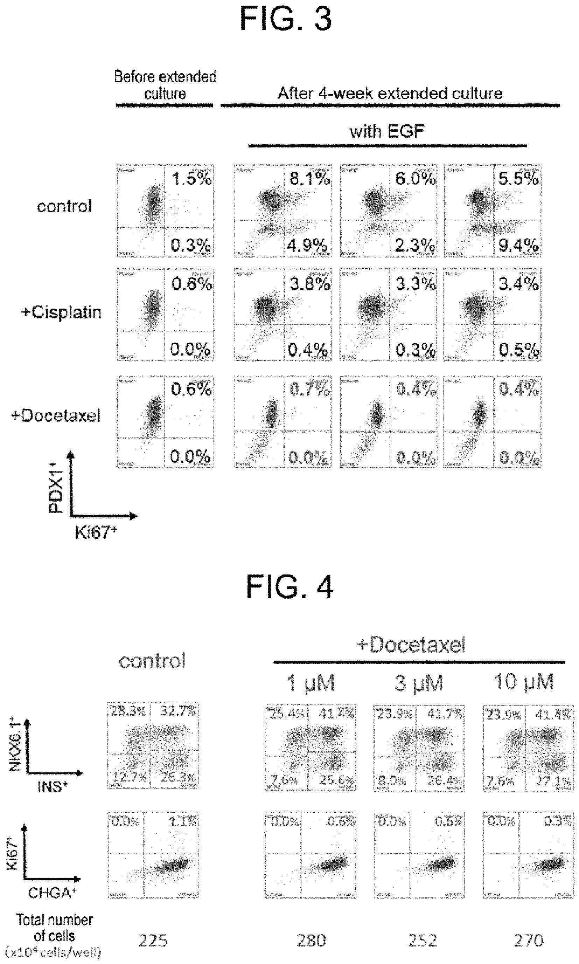

[Figure 3] Figure 3 shows results of extended-culturing an insulin-producing cell population obtained by treatment with cisplatin or docetaxel in a basal medium containing EGF, and then analyzing the expression of marker proteins (PDX1 and Ki67) in each resulting cell population by flow cytometry. The control cell population is an insulin-producing cell population obtained without the use of any of cisplatin and docetaxel, and was extended-cultured in the same manner as above.
[Figure 4] Figure 4 shows results of analyzing the expression of marker proteins (INS and NKX6.1; and CHGA and Ki67) by flow cytometry in an insulin-producing cell population obtained by treatment with docetaxel. The control cell population is an insulin-producing cell population obtained without the use of docetaxel.

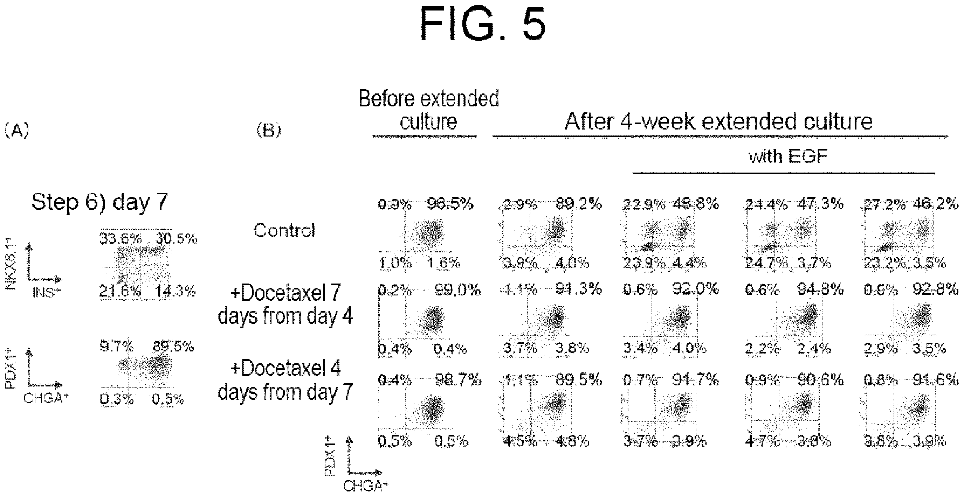

[Figure 5] Figure 5 shows (A) results of analyzing the expression of marker proteins (NKX6.1, C-peptide (insulin), PDX1, and CHGA) by flow cytometry in a cell population obtained 7 days after the start of step 6), and (B) results of extended-culturing an insulin-producing cell population obtained by treatment with docetaxel in a basal medium containing EGF for 7 days from 4 days after the start of step 6) or 4 days from 7 days after the start of step 6), and then analyzing the expression of marker proteins (PDX1 and CHGA) in each resulting cell population by flow cytometry. The control cell population is an insulin-producing cell population obtained without the use of docetaxel, and was extended-cultured in the same manner as above.

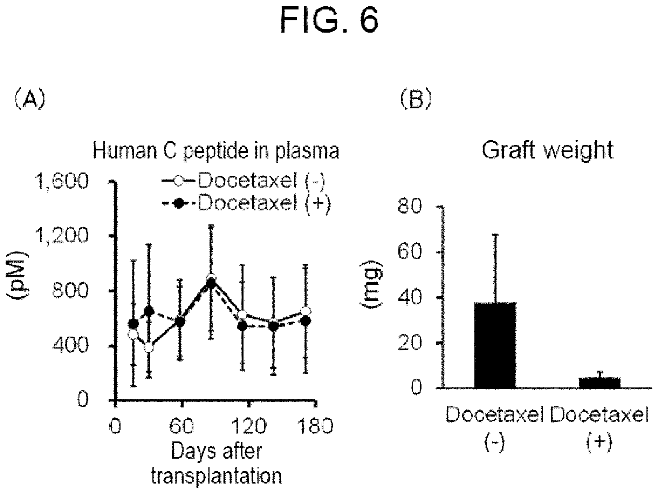

[Figure 6] Figure 6 shows results of measurement of (A) a human C-peptide concentration in plasma in an immunodeficient NOD/SCID mouse induced to have insulin-deficient diabetes mellitus in which an insulin-producing cell population obtained by treatment with docetaxel (docetaxel(+)) or an insulin-producing cell population prepared without the addition of docetaxel (docetaxel(-)) was subcutaneously transplanted, and (B) a weight of the graft excised 6 month after transplantation.

### Description of Embodiments

### 1. Terminology

Hereinafter, the terms described herein will be described.

As used herein, "about" or "around" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

As used herein, "comprise(s)" or "comprising" means inclusion of the element(s) following the word without limitation thereto. Accordingly, it indicates inclusion of the element(s) following the word, but does not indicate exclusion of any other element.

As used herein, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

As used herein, "without the use of feeder cell(s)" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

As used herein, "adhered (adherent)" refers to cells are attached to a container, for example, cells are attached to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

As used herein, "culture" refers to maintaining, growing, and/or differentiating cells in in vitro environment. "Culturing" means maintaining, proliferating, and/or differentiating cells out of tissue or the living body, for example, in a cell culture dish or flask. The culture includes two-dimensional culture (plane culture) and three-dimensional culture (suspension culture).

As used herein, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

As used herein, "deplete(s)" and "depletion" refer to decreasing the amount of a certain component in a composition such as a composition of cells and "depleted" refers, when used to describe a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

As used herein, "purify(ies)" and "purification" refer to removing impurities in a composition such as a composition of cells and making it pure for a certain component and "purified" refers, when used to describe a composition of cells, for example, a cell population, to a cell population in which the amount of impurities is decreased in comparison with the percentage of such components in the cell population before purification and the purity of a certain component is improved. For example, a composition such as a cell population can be purified for a target cell type and, accordingly, the percentage of the target cell type is increased in comparison with the percentage of the target cells present in the cell population before the purification. A cell population can be purified for a target cell type by a method of selecting and sorting cells known in the art. A cell population can be purified by a specific process of sorting or selection described herein. In a certain embodiment of the present invention, the purity of a target cell population is brought by a method of purifying a target cell population to at least 70%, 80%, 85%, 90%, 95%, 97%, 98%, or 99% or to the extent at which impurities (including contaminant cells) are undetectable.

As used herein, "marker" means a cell antigen or a gene thereof that is specifically expressed depending on a predetermined cell type, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, "positive" for a marker protein means being detected to be positive by flow cytometry and "negative" therefor means being equal to or less than the lower detection limit in flow cytometry. Also, as used herein, "positive" for a marker gene means being detected by RT-PCR and "negative" therefor means being equal to or less than the lower detection limit in RT-PCR.

As used herein, "expression" is defined as transcription and/or translation of a certain nucleotide sequence driven by an intracellular promoter.

As used herein, "factor having CDK8/19-inhibiting activity" means any substance having the inhibitory activity for CDK8/19. CDK8, in contrast to the other proteins of the same CDK family, is not required for cell proliferation. The inhibition of CDK8 has no great effect under usual conditions. CDK19 and CDK8 are similar to each other. Usually, the inhibition of CDK8 also involves the inhibition of CDK19. In the present invention, conventionally known factor having CDK8/19-inhibiting activity can be used, and such a factor having CDK8/19-inhibiting activity can be found from patent literatures or non patent literatures. Examples thereof include, among compounds described in US2012/0071477, WO2015/159937, WO2015/159938, WO2013/116786, WO2014/0038958, WO2014/134169, JP2015/506376, US2015/0274726, US2016/0000787, WO2016/009076, WO2016/0016951, WO2016/018511, WO2016/100782 and WO2016/182904, compounds (and salts thereof) having CDK8/19-inhibiting activity. More specific examples of the factor having CDK8/19-inhibiting activity include diethyl (E)-(4-(3-(5-(4-fluorophenyl)-1-methyl-1H-pyrazol-4-yl)acrylamido)benzyl)phosphonate, 2-(4-(4-(isoquinolin-4-yl)phenyl)-1H-pyrazol-1-yl)-N,N-dimethylacetamide, 4-((2-(6-(4-methylpiperazine-1-carbonyl)naphthalen-2-yl)ethyl)amino)quinazoline-6-carbonitrile, 4-(4-(2,3-dihydrobenzo[b][1,4]dioxin-6-yl)-1H-pyrazol-3-yl)benzene-1,3-diol, 3-(2-(imidazo[1,2-b]pyridazin-6-ylthio)ethyl)-4-(naphthalen-1-ylsulfornyl)-3,4-dihydroquinoxalin-2(1H)-one, (E)-3-(4-(1-cyclopropyl-1H-pyrazol-4-yl)pyridin-3-yl)-N-(4-(morpholinomethyl)phenyl)acrylamide, 8-(2,4-difluorophenoxy)-1-methyl-4,5-dihydro-1H-thieno[3,4-g]indazole-6-carboxamide, 4-((4-fluorophenyl)sulfonyl)-3-(2-(imidazo[1,2-b]pyridazin-6-ylsulfanyl)ethyl)-3,4-dihydroquinoxalin-2(1H)-one, 2-(benzylamino)-4-(1H-pyrrolo[2,3-b]pyridin-3-yl)benzamide, 3-(3-(benzyloxy)phenyl)-1H-pyrrolo[2,3-b]pyridine, 4-(4-(2,3-dihydro-1,4-benzodioxin-6-yl)-1H-pyrazol-3-yl)benzene-1,3-diol, N-butyl-8-(4-methoxyphenyl)-1,6-naphthyridine-2-carboxamide, 8-(4-methylphenyl)-N,N-dipropyl-1,6-naphthyridine-2-carboxamide and salts thereof.

The factor having CDK8/19-inhibiting activity is not limited to the compounds described above, and an antisense oligonucleotide or siRNA against CDK8/19 mRNA, an antibody that binds to CDK8/19, a dominant negative CDK8/19 mutant, or the like can also be used as the CDK8/19 inhibitor. The factor having CDK8/19-inhibiting activity or the CDK8/19 inhibitor is commercially available or can be synthesized for use according to a known method.

As used herein, "growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony stimulating factors (for example, granulocyte/macrophage-colony stimulating factor (GM-CSF)), various interferons (for example, IFN-y, and the like), and other cytokines having effects on stem cells, for example, stem cell factor (SCF), and erythropoietin (Epo).

As used herein, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-1α-carbamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{ [2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used as the ROCK inhibitors. The ROCK inhibitors are commercially available or can be synthesized according to a known method.

As used herein, "GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. The GSK3β inhibitors are not limited to these and antisense oligonucleotides and siRNA to GSK3β mRNA, antibodies that bind to GSK3β, dominant negative GSK3β mutants, and the like can also be used as the GSK3β inhibitors. The GSK3β inhibitors are commercially available, or can be synthesized according to a known method.

As used herein, "FGFR1 inhibitors" are substances having the inhibitory activity at least for fibroblast growth factor receptor (FGFR) 1. FGFR1 is a member of the four-pass transmembrane tyrosine kinase family (FGFR1,2,3,4) as a receptor having high affinity for growth factors FGF1 to FGF17. As used herein, "FGFR1 inhibitors" can have at least FGFR1-inhibiting activity and may have the inhibitory activity for other FGFRs. As used herein, "FGFR1 inhibitors" may be, for example, FGFR2 inhibitors, FGFR3 inhibitors, or FGFR4 inhibitors as long as these inhibitors have FGFR1-inhibiting activity. In the present invention, conventionally known FGFR1 inhibitors can be used. More specific examples of the FGFR1 inhibitors include PD-166866 (1-[2-amino-6-(3,5-dimethoxyphenyl)-pyrido(2,3-d)pyrimidin-7-yl]-3-tert-butylurea: CAS No.: 192705-79-6), E-3810 (CAS No.: 1058137-23-7), PD-173074 (CAS No.: 219580-11-7), FGFR4-IN-1 (CAS No.: 1708971-72-5), FGFR-IN-1 (CAS No.: 1448169-71-8), FIIN-2 (CAS No.: 1633044-56-0), AZD4547 (CAS No.: 1035270-39-3), FIIN-3 (CAS No.: 1637735-84-2), NVP-BGJ398 (CAS No.: 1310746-10-1), NVP-BGJ398 (CAS No.: 872511-34-7), CH5183284 (CAS No.: 1265229-25-1), Derazantinib (CAS No.: 1234356-69-4), Derazantinib Racemate, Ferulic acid (CAS No.: 1135-24-6), SSR128129E (CAS No.: 848318-25-2), SSR128129E free acid (CAS No.: 848463-13-8), Erdafitinib (CAS No.: 1346242-81-6), BLU9931 (CAS No.: 1538604-68-0), PRN1371 (CAS No.: 1802929-43-6), S49076 (CAS No.: 1265965-22-7), LY2874455 (CAS No.: 1254473-64-7), Linsitinib (CAS No.: 867160-71-2), Dovitinib (CAS No.: 405169-16-6), Anlotinib (CAS No.: 1058156-90-3), Brivanib (CAS No.: 649735-46-6), Derazantinib (CAS No.: 1234356-69-4), Anlotinib Dihydrochloride (CAS No.: 1360460-82-7), ACTB-1003 (CAS No.: 939805-30-8), BLU-554 (CAS No.: 1707289-21-1), Rogaratinib (CAS No.: 1443530-05-9), BIBF 1120 esylate (CAS No.: 656247-18-6), TG 100572 Hydrochloride (CAS No.: 867331-64-4), ENMD-2076 (CAS No.: 934353-76-1), Brivanib alaninate (CAS No.: 649735-63-7), TG 100572 (CAS No.: 867334-05-2), BIBF 1120 (CAS No.: 656247-17-5), ENMD-2076 Tartrate (CAS No.: 1291074-87-7), TSU-68 (CAS No.: 252916-29-3), Ponatinib (CAS No.: 943319-70-8), Sulfatinib (CAS No.: 1308672-74-3), LY2784544 (CAS No.: 1229236-86-5), Dovitinib lactate (CAS No.: 692737-80-7), SU 5402 (CAS No.: 215543-92-3), FGF-401 (CAS No.: 1708971-55-4), Tyrosine kinase-IN-1 (CAS No.: 705946-27-6), PP58 (CAS No.: 212391-58-7), TG 100801 Hydrochloride (CAS No.: 1018069-81-2), Crenolanib (CAS No.: 670220-88-9), TG 100801 (CAS No.: 867331-82-6), Pazopanib Hydrochloride (CAS No.: 635702-64-6), Pazopanib (CAS No.: 444731-52-6), PD168393 (CAS No.: 194423-15-9), Apatinib (CAS No.: 1218779-75-9), Palbociclib isethionate (CAS No.: 827022-33-3), Foretinib (CAS No.: 849217-64-7), Lenvatinib (CAS No.: 417716-92-8), Tandutinib (CAS No.: 387867-13-2) and salts thereof.

The FGFR1 inhibitors are not limited to the compounds described above, and antisense oligonucleotides and siRNA to FGFR1 mRNA, antibodies that bind to FGFR1, and dominant negative FGFR1 mutants can also be used as the FGFR1 inhibitors. The FGFR1 inhibitors are commercially available or can be synthesized according to a known method.

As used herein, examples of "serum replacement" include KnockOut(TM) Serum Replacement (KSR: Thermo Fisher Scientific), StemSure(R) Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure(R) Serum Replacement, ITS-G. The concentration of serum replacement in a medium when added into a medium is 0.01-10% by weight, and preferably 0.1-2% by weight. In the present invention, "serum replacement" is preferably used instead of serum.

### 2. Method for producing insulin-producing cell population, and insulin-producing cell population produced thereby

The present invention provides a method for producing an insulin-producing cell population, comprising treating a pancreatic endocrine progenitor cell population and/or a cell population at a later stage of differentiation with a microtubule inhibitor in the process of inducing the differentiation of a pancreatic endocrine progenitor cell population and/or a cell population at a later stage of differentiation, obtained by the induction of differentiation from pluripotent stem cells into an insulin-producing cell population, and an insulin-producing cell population prepared thereby.

In the present invention, the microtubule inhibitor in the process of inducing the differentiation of a pancreatic endocrine progenitor cell population and/or a cell population at a later stage of differentiation, obtained by the induction of differentiation from pluripotent stem cells into an insulin-producing cell population acts on the pancreatic endocrine progenitor cell population and/or the cell population at a later stage of differentiation to give rise to an insulin-producing cell population in which endogenous non-endocrine cells have been reduced.

In the present invention, herein, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

In the present invention, "multipotency" means the ability to differentiate into plural and limited numbers of linages of cells. For example, mesenchymal stem cells, hematopoietic stem cells, neural stem cells are multipotent, but not pluripotent.

In the present invention, "pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, preferably, pluripotent stem cells are human pluripotent stem cells.

Available "ES cells" include murine ES cells, such as various murine ES cell lines established by inGenious, Institute of Physical and Chemical Research (RIKEN), and the like, and human ES cells, such as various human ES cell lines established by National Institutes of Health (NIH), RIKEN, Kyoto University, Cellartis, and the like. For example, available ES cell lines include CHB-1 to CHB-12, RUES1, RUES2, HUES1 to HUES28 from NIH, and the like; H1 and H9 from WiCell Research Institute; and KhES-1, KhES-2, KhES-3, KhES-4, KhES-5, SSES1, SSES2, SSES3 from RIKEN, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, and c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, Institute of Physical and Chemical Research (RIKEN), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, CDI cell lines MyCell iPS Cells (21525.102.10A), MyCell iPS Cells (21526.101.10A), and the like.

In the present invention, "pancreatic endocrine progenitor cell population" means a cell population comprising pancreatic endocrine progenitor cells. In the present invention, pancreatic endocrine progenitor cells mean cells characterized by the expression of at least one of the markers chromogranin A (CHGA), NeuroD and NGN3 and no expression of a marker of the pancreas-related hormone system (for example, insulin). The pancreatic endocrine progenitor cells may express a marker such as PAX-4, NKX2.2, Islet-1, or PDX-1.

In one embodiment, "pancreatic endocrine progenitor cell population" according to the present invention is a cell population that corresponds to a culture after the completion of step 5) or a culture in step 6) in the process of inducing the differentiation of pluripotent stem cells into insulin-producing cells as described below in detail.

"Pancreatic endocrine progenitor cell population" according to the present invention comprises pancreatic endocrine progenitor cells at a proportion of 30% or more, preferably 40% or more, more preferably 50% or more, further preferably 60% or more, still further preferably 70% or more. The upper limit of the proportion is not particularly limited and is 90% or less, 80% or less, 70% or less, or 60% or less. The proportion can be expressed using two numeric values respectively selected from the numeric values of the upper limit and the lower limit. The proportion is, for example, 30% to 90%, preferably 40% to 80%, more preferably 50% to 80%, further preferably 60% to 70%. The pancreatic endocrine progenitor cell population may include other cells (for example, pancreatic progenitor cells, insulin-producing cells, Ki67-positive cells, and CHGA-negative cells), in addition to the pancreatic endocrine progenitor cells.

In the present invention, "cell population at a later stage of differentiation" means a cell population richer in cells whose stage of differentiation is more advanced than that of pancreatic endocrine progenitor cells, as compared with a pancreatic endocrine progenitor cell population. Examples of "cells whose stage of differentiation more advanced than that of pancreatic endocrine progenitor cells" include insulin-producing cells. In the present invention, insulin-producing cells mean cells characterized by the expression of at least one of the markers insulin and NKX6.1, preferably the expression of both the markers insulin and NKX6.1. The expression level of NGN3 in the insulin-producing cells is preferably at a proportion of less than 1/3 of the maximum expression confirmed in pancreatic endocrine progenitor cells.

"Cell population at a later stage of differentiation" according to the present invention comprises insulin-producing cells at a proportion of 30% or more, preferably 40% or more, more preferably 50% or more, further preferably 60% or more, still further preferably 70% or more. The upper limit of the proportion is not particularly limited and is 90% or less, 80% or less, 70% or less, or 60% or less. The proportion can be expressed using two numeric values respectively selected from the numeric values of the upper limit and the lower limit. The proportion is, for example, 30% to 90%, preferably 40% to 80%, more preferably 50% to 80%, further preferably 60% to 70%. "Cell population at a later stage of differentiation" may include other cells (for example, pancreatic endocrine progenitor cells, insulin-producing cells, Ki67-positive cells, and CHGA-negative cells), in addition to the insulin-producing cells.

In one embodiment, "cell population at a later stage of differentiation" according to the present invention corresponds to a cell population of a culture from a period of 3 days, 4 days, 5 days, 6 days, 7 days, 8 days or more before the completion of step 6) or a culture after the completion of step 6) in the process of inducing the differentiation of pluripotent stem cells into insulin-producing cells as described below in detail.

The proportion of specific cells in a cell population described herein can be determined on the basis of a known approach capable of calculating the number of cells, such as flow cytometry.

It is known that cells having different features depending on the stages of differentiation appear in the process of differentiation of pluripotent stem cells into insulin-producing cells (WO2009/012428 and WO2016/021734). For example, the stages of differentiation can be broadly classified into pluripotent stem cells, definitive endoderm cells, primitive gut tube cells, posterior foregut cells, pancreatic progenitor cells, pancreatic endocrine progenitor cells, and insulin-producing cells in order from relatively undifferentiated to differentiated forms.

The pancreatic endocrine progenitor cell population can be obtained by use of the following known process of inducing differentiation which involves inducing the differentiation of pluripotent stem cells into definitive endoderm cells, primitive gut tube cells, posterior foregut cells, pancreatic progenitor cells, pancreatic endocrine progenitor cells, and insulin-producing cells:
step 1) inducing the differentiation of pluripotent stem cells into definitive endoderm cells;
step 2) inducing the differentiation of the definitive endoderm cells into primitive gut tube cells;
step 3) inducing the differentiation of the primitive gut tube cells into posterior foregut cells;
step 4) inducing the differentiation of the posterior foregut cells into pancreatic progenitor cells;
step 5) inducing the differentiation of the pancreatic progenitor cells into pancreatic endocrine progenitor cells; and
step 6) inducing the differentiation of the pancreatic endocrine progenitor cells into insulin-producing cells.

Hereinafter, each step will be described, though the induction of differentiation into each cell is not limited by these approaches.

### Step 1) Differentiation into definitive endoderm cells

The pluripotent stem cells are cultured in a medium containing a low dose of activin A to thereby differentiate into definitive endoderm cells.

The medium used in this step may be a basal medium for use in the culture of mammalian cells, such as RPMI medium, MEM medium, iMEM medium, DMEM (Dulbecco's modified Eagle medium) medium, Improved MEM Zinc Option medium, Improved MEM/1% B-27 supplement/Penicillin Streptomycin medium, or MCDB131/10 mM Glucose/20 mM Glucose/NaHCO₃/FAF-BSA/ITS-X/Glutamax/ascorbic acid/Penicillin Streptomycin medium.

The activin A can be contained at a low dose, for example, in an amount of 5 to 100 ng/mL, preferably 5 to 50 ng/mL, more preferably 5 to 10 ng/mL, in the medium.

In another embodiment, the concentration of the activin A in the medium is about 0.1 to 100 ng/mL, preferably about 1 to 50 ng/mL, more preferably about 3 to 10 ng/mL.

The medium can be further supplemented with a ROCK inhibitor and/or a GSK3β inhibitor.

The concentration of the GSK3β inhibitor in the medium is appropriately set depending on the type of the GSK3β inhibitor used. For example, in the case of using CHIR99021 as the GSK3β inhibitor, its concentration is usually 2 to 5 µM, preferably 2 to 4 µM, particularly preferably about 3 µM.

The concentration of the ROCK inhibitor in the medium is appropriately set depending on the type of the ROCK inhibitor used. For example, in the case of using Y27632 as the ROCK inhibitor, its concentration is usually 5 to 20 µM, preferably 5 to 15 µM, particularly preferably about 10 µM.

The medium can be further supplemented with insulin. The insulin can be contained in an amount of 0.01 to 20 µM, preferably 0.1 to 10 µM, more preferably 0.5 to 5 µM, in the medium. The concentration of the insulin in the medium may be, but is not limited to, the concentration of insulin contained in added B-27 supplement.

The culture may be performed by any of two-dimensional culture and three-dimensional culture. The number of cells at the start of culture is not particularly limited and can be about 50000 to 1000000 cells/cm², preferably about 100000 to 800000 cells/cm², more preferably about 100000 to 300000 cells/cm², for two-dimensional culture. The number of cells at the start of culture is not particularly limited and can be about 10000 to 1000000 cells/mL, preferably about 100000 to 800000 cells/mL, more preferably about 300000 to 600000 cells/mL, for three-dimensional culture. The culture period is 1 day to 4 days, preferably 1 day to 3 days, particularly preferably 3 days.

The culture temperature is not particularly limited, and the culture is performed at 30 to 40°C (for example, 37°C). The concentration of carbon dioxide in a culture container is on the order of, for example, 5%.

Alternatively, the definitive endoderm cells according to the present invention can be produced by subjecting the pluripotent stem cells to first culture in a medium under conditions causing action of insulin in the presence of a low dose of activin A and subsequently to second culture in a medium under conditions causing no action of insulin.

### (1) First culture

"Conditions causing action of insulin" mean conditions that activate an insulin signal transduction pathway in cells by insulin. Usually, insulin binds to an insulin receptor present on cell membrane surface so that tyrosine kinase incorporated in the receptor is activated for the tyrosine phosphorylation of the insulin receptor substrate protein family (IRS: IRS-1,2,3). As used herein, "activating an insulin signal transduction pathway" refers to causing this series of reactions initiated by the binding of insulin to an insulin receptor.

Examples of the conditions causing action of insulin include the case where the medium contains insulin. The insulin can be insulin that can activate an insulin signal transduction pathway in pluripotent stem cells, and may be produced by a recombination method or may be produced through synthesis by a solid-phase synthesis method. Insulin derived from a human, a nonhuman primate, a pig, cattle, a horse, sheep, a goat, a llama, a dog, a cat, a rabbit, a mouse, a guinea pig, or the like can be used. Human insulin is preferred.

In the present invention, an insulin mutant, an insulin derivative or an insulin agonist may be used as "insulin" as long as it can activate an insulin signal transduction pathway in pluripotent stem cells. Examples of "insulin mutant" include ones having a polypeptide that consists of an amino acid sequence derived from the amino acid sequence of insulin by the deletion, substitution, addition or insertion of 1 to 20, preferably 1 to 10, more preferably 1 to 5 amino acids and is capable of activating an insulin signal transduction pathway, or a polypeptide that consists of an amino acid sequence having 80% or more, preferably 90% or more, more preferably 95% or more, most preferably 99% or more sequence identity to the amino acid sequence of insulin and is capable of activating an insulin signal transduction pathway. Amino acid sequences can be compared by a known approach. The comparison can be carried out using, for example, BLAST (Basic Local Alignment Search Tool at the National Center for Biological Information), for example, at default settings. "Insulin derivative" means a polypeptide that consists of an amino acid sequence obtained by the chemical substitution (for example, α-methylation, α-hydroxylation), deletion (for example, deamination), or modification (for example, N-methylation) of one or some of groups of amino acid residues of insulin or an insulin mutant, and is capable of activating an insulin signal transduction pathway, or a substance having a similar effect. "Insulin agonist" means a polypeptide capable of activating an insulin signal transduction pathway by binding to an insulin receptor or a substance having a similar effect, regardless of the structure of insulin.

The medium for the first culture can contain the insulin in an amount of 0.01 to 20 µM, preferably 0.1 to 10 µM, more preferably 0.5 to 5 µM. The concentration of the insulin in the medium may be the concentration of insulin contained in added B-27 supplement, but is not limited thereto. As used herein, B-27 supplement containing insulin is also referred to as "B-27(INS+)", and B-27 supplement containing no insulin is also referred to as "B-27(INS-)".

The medium can further contain a ROCK inhibitor and/or a GSK3β inhibitor. The concentration of the ROCK inhibitor in the medium is appropriately set depending on the type of the ROCK inhibitor used. For example, in the case of using Y27632 as the ROCK inhibitor, its concentration can be usually 5 to 20 µM, preferably 5 to 15 µM, particularly preferably about 10 µM. The concentration of the GSK3β inhibitor in the medium is appropriately set depending on the type of the GSK3β inhibitor used. For example, in the case of using CHIR99021 as the GSK3β inhibitor, its concentration can be usually 2 to 5 µM, preferably 2 to 4 µM, particularly preferably about 3 µM.

The medium can further contain one or more members selected from the group consisting of pyruvate (sodium salt, etc.), L-alanyl L-glutamine, and glucose. The pyruvate can be contained in an amount of 10 to 1000 mg/L, preferably 30 to 500 mg/L, more preferably 50 to 200 mg/L, particularly preferably about 110 mg/L, in the medium. The L-alanyl L-glutamine can be contained in an amount of 50 to 2000 mg/L, preferably 100 to 1500 mg/L, more preferably 500 to 1000 mg/L, particularly preferably about 860 mg/L, in the medium. The glucose can be contained in an amount of 15 mM or more, preferably 15 to 30 mM, more preferably 15 to 25 mM, particularly preferably about 25 mM, in the medium. The concentrations of the pyruvate, the L-alanyl L-glutamine and the glucose in the medium may be the concentrations of pyruvate, L-alanyl L-glutamine and glucose contained in DMEM medium (DMEM, high glucose, GlutaMAX(TM), pyruvate (Thermo Fisher Scientific)) or other DMEM media, but are not limited thereto.

The medium is based on the basal medium and can be supplemented with one or more of the components described above for use. The basal medium is preferably DMEM medium, more preferably DMEM medium containing pyruvate, L-alanyl L-glutamine, and glucose in the amounts described above.

The culture period of the first culture can be in a range selected from 6 hours to 48 hours, preferably 12 to 24 hours. The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture. The number of cells at the start of culture is not particularly limited and can be about 50000 to 1000000 cells/cm², preferably about 100000 to 800000 cells/cm², more preferably about 100000 to 300000 cells/cm², for two-dimensional culture. The number of cells at the start of culture is not particularly limited and can be about 10000 to 1000000 cells/mL, preferably about 100000 to 800000 cells/mL, more preferably about 300000 to 600000 cells/mL, for three-dimensional culture.

### (2) Second culture

"Conditions causing no action of insulin" mean conditions that do not activate an insulin signal transduction pathway in cells by insulin. "Not activate an insulin signal transduction pathway in cells" not only means that there occurs no activation of the insulin signal transduction pathway but means that there occurs slight activation to an extent that no significant difference is found as compared with the activation of the insulin signal transduction pathway in the absence of insulin. Thus, examples of "conditions causing no action of insulin" include the absence of insulin in the medium, and, even if insulin is contained in the medium, conditions where the insulin is contained in an amount that causes the slight activation to an extent that no significant difference is found. Alternatively, "conditions causing no action of insulin" also mean that, even if insulin is contained in the medium, the insulin signal transduction pathway is not activated owing to the coexistence of an insulin signaling inhibitor. "Insulin signaling inhibitor" means a component capable of blocking an insulin signal transduction pathway at any position. Examples of such an insulin signaling inhibitor include polypeptides and compounds that bind to or compete with various proteins or the like acting as insulin, an insulin receptor, or a signal transducer and thereby inhibit intermolecular interaction involving these factors. Examples of such an insulin signaling inhibitor include LY294002 [2-(4-morpholinyl)-8-phenyl-4H-1-benzopyran-4-one] which competitively inhibits ATP binding to a catalytic subunit of PI3 kinase. The insulin signaling inhibitor is not limited to these, and, for example, an antibody that binds to any of various proteins acting as insulin, an insulin receptor, or a signal transducer, a dominant negative mutant thereof, or an antisense oligonucleotide or siRNA against mRNA of any of various proteins acting as an insulin receptor or a signal transducer can also be used as the insulin signaling inhibitor. The insulin signaling inhibitor is commercially available or can be synthesized according to a known method.

The medium can further contain a ROCK inhibitor and/or a GSK3β inhibitor. The amount of the ROCK inhibitor and/or the GSK3β inhibitor in the medium can be selected from the range described about the first culture and may be the same as or different from the amount used for the first culture.

The medium can further contain one or more members selected from the group consisting of pyruvate, L-alanyl L-glutamine, and glucose. The amounts of the pyruvate, the L-alanyl L-glutamine, and the glucose in the medium can be selected from the ranges described about the first culture and may be the same as or different from the amounts used for the first culture.

The medium for use in the second culture is based on a basal medium for use in the culture of mammalian cells and can be supplemented with one or more of the components described above for use. The basal medium described about the first culture can be used, and the basal medium may be the same as or different from that used for the first culture. DMEM medium is preferred, and DMEM medium containing pyruvate, L-alanyl L-glutamine, and glucose in the amounts described above is more preferred.

The culture period of the second culture is at least 6 hours and can be in a range selected from preferably 6 to 72 hours, more preferably 24 hours to 72 hours. The culture temperature is not particularly limited, and the culture is performed at 30 to 40°C (for example, 37°C). The culture may be performed by any of two-dimensional culture and three-dimensional culture. The concentration of carbon dioxide in a culture container is on the order of, for example, 5%.

The media for the first culture and the second culture can contain the low dose of activin A. The amount of activin A contained may be the same or different between the media for the first culture and the second culture.

The media for the first culture and the second culture can further contain dimethyl sulfoxide.

The proportion of the resulting endocrine cells in step 6) or later can be enhanced by culturing the pluripotent stem cells in the presence of a low dose of activin A or by subjecting the pluripotent stem cells to first culture in a medium under conditions causing action of insulin in the presence of a low dose of activin A and subsequently to second culture in a medium under conditions causing no action of insulin.

### Step 2) Differentiation into primitive gut tube cells

The definitive endoderm cells obtained in step 1) are further cultured in a medium containing a growth factor to induce their differentiation into primitive gut tube cells. The culture period is 2 days to 8 days, preferably about 4 days.

The culture temperature is not particularly limited, and the culture is performed at 30 to 40°C (for example, 37°C). The concentration of carbon dioxide in a culture container is on the order of, for example, 5%. The culture may be performed by any of two-dimensional culture and three-dimensional culture.

A basal medium for use in the culture of mammalian cells described in step 1) can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

### Step 3) Differentiation into posterior foregut cells

The primitive gut tube cells obtained in step 2) are further cultured in a medium containing a growth factor, cyclopamine, noggin, and the like to induce their differentiation into posterior foregut cells. The culture period is 1 day to 5 days, preferably about 2 days. The culture may be performed by any of two-dimensional culture and three-dimensional culture.

The culture temperature is not particularly limited, and the culture is performed at 30 to 40°C (for example, 37°C). The concentration of carbon dioxide in a culture container is on the order of, for example, 5%.

The basal medium for use in the culture of mammalian cells described in step 1) can be used as culture medium. The medium may be appropriately supplemented with a serum replacement, a vitamin, an antibiotic, and the like, in addition to the growth factor.

The growth factor is preferably EGF, KGF, and/or FGF10, more preferably EGF and/or KGF, further preferably KGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF as the growth factor, its concentration is usually 5 to 150 ng/mL, preferably 30 to 100 ng/mL, particularly preferably about 50 ng/mL.

The concentration of the cyclopamine in the medium is not particularly limited and is usually 0.5 to 1.5 µM, preferably 0.3 to 1.0 µM, particularly preferably about 0.5 µM.

The concentration of the noggin in the medium is not particularly limited and is usually 10 to 200 ng/mL, preferably 50 to 150 ng/mL, particularly preferably about 100 ng/mL.

The medium may be supplemented with dimethyl sulfoxide.

### Step 4) Differentiation into pancreatic progenitor cells

The posterior foregut cells obtained in step 3) may be further cultured in a medium containing a factor having CDK8/19-inhibiting activity, preferably a medium containing a factor having CDK8/19-inhibiting activity and a growth factor, to induce their differentiation into pancreatic progenitor cells. The culture period is 2 days to 10 days, preferably about 5 days. The culture may be performed by any of two-dimensional culture and three-dimensional culture. In the case of two-dimensional culture, according to the previous report (Toyoda et al., Stem cell Research (2015) 14, 185-197), the posterior foregut cells obtained in step 3) are treated with 0.25% trypsin-EDTA and then dispersed by pipetting, and the obtained dispersion is subjected to centrifugal separation. Recovered cells are resuspended in a fresh medium of step 4) and the cell suspension is reseeded to a fresh two-dimensional culture container.

The basal medium for use in the culture of mammalian cells described in step 1) can be used as culture medium. The medium may be appropriately supplemented with a serum replacement, a vitamin, an antibiotic, and the like, in addition to the growth factor.

Each of the compounds mentioned above or salts thereof can be used as the factor having CDK8/19-inhibiting activity. The amount of the factor added to the medium is appropriately determined according to the compound or the salt thereof used and is usually about 0.00001 µM to 5 µM, preferably 0.00001 µM to 1 µM. The concentration of the factor having CDK8/19-inhibiting activity in the medium is preferably a concentration that attains inhibitory activity of 50% or more for CDK8/19.

The growth factor is preferably EGF, KGF, and/or FGF10, more preferably KGF and/or EGF, further preferably KGF and EGF.

The concentration of the growth factor in the medium is appropriately set depending on the type of the growth factor used and is usually about 0.1 nM to 1000 µM, preferably about 0.1 nM to 100 µM. In the case of EGF, its concentration is about 5 to 2000 ng/ml (that is, about 0.8 to 320 nM), preferably about 5 to 1000 ng/ml (that is, about 0.8 to 160 nM), more preferably about 10 to 1000 ng/ml (that is, about 1.6 to 160 nM). In the case of FGF10, its concentration is about 5 to 2000 ng/ml (that is, about 0.3 to 116 nM), preferably about 10 to 1000 ng/ml (that is, about 0.6 to 58 nM), more preferably about 10 to 1000 ng/ml (that is, about 0.6 to 58 nM). For example, in the case of using KGF and EGF as the growth factor, the concentration of EGF is usually 5 to 150 ng/mL, preferably 30 to 100 ng/mL, particularly preferably about 50 ng/mL, and the concentration of KGF is usually 10 to 200 ng/mL, preferably 50 to 150 ng/mL, particularly preferably about 100 ng/mL.

Culture on the first day in step 4) may be performed in the presence of a ROCK inhibitor, and culture on the following days may be performed in a medium containing no ROCK inhibitor.

The medium may also contain a PKC activator. PdBU (PKC activator II), TPB (PKC activator V), or the like is used as the PKC activator, though the PKC activator is not limited thereto. The concentration of the PKC activator to be added is about 0.1 to 100 ng/ml, preferably about 1 to 50 ng/ml, more preferably about 3 to 10 ng/ml.

The medium may also be supplemented with dimethyl sulfoxide and/or activin (1 to 50 ng/ml).

In any of the steps, the medium may be supplemented with a serum replacement (for example, B-27 supplement, ITS-G), in addition to the components described above. Also, an amino acid, L-glutamine, GlutaMAX (product name), a non-essential amino acid, a vitamin, nicotinamide, an antibiotic (for example, Antibiotic-Antimycotic (also referred as AA herein), penicillin, streptomycin, or a mixture thereof), an antimicrobial agent (for example, amphotericin B), an antioxidant, pyruvic acid, a buffer, inorganic salts, and the like may be added thereto, if necessary. In the case of adding an antibiotic to the medium, its concentration in the medium is usually 0.01 to 20% by weight, preferably 0.1 to 10% by weight. The culture may be performed by any of two-dimensional culture and three-dimensional culture.

In the case of two-dimensional culture, the cell culture is performed by adherent culture without the use of feeder cells. For the culture, a culture container, for example, a dish, a flask, a microplate, or a cell culture sheet such as OptiCell (product name) (Nunc), is used. The culture container is preferably surface-treated in order to improve adhesiveness to cells (hydrophilicity), or coated with a substrate for cell adhesion such as collagen, gelatin, poly-L-lysine, poly-D-lysine, laminin, fibronectin, Matrigel (for example, BD Matrigel (Nippon Becton Dickinson Company, Ltd.)), or vitronectin. The culture container is preferably a culture container coated with type I-collagen, Matrigel, fibronectin, vitronectin or poly-D-lysine, more preferably a culture container coated with Matrigel or poly-D-lysine.

The culture temperature is not particularly limited, and the culture is performed at 30 to 40°C (for example, 37°C). The concentration of carbon dioxide in a culture container is on the order of, for example, 5%.

The pancreatic progenitor cells obtained in step 4) can be further purified using a known surface marker glycoprotein 2 (GP2) or the like. The purification can be performed by a method known per se, for example, using anti-GP2 antibody-immobilized beads.

### Step 5) Differentiation into pancreatic endocrine progenitor cells

The pancreatic progenitor cells obtained in step 4) are further cultured in a medium containing a growth factor to induce their differentiation into pancreatic endocrine progenitor cells. The culture may be performed by any of two-dimensional culture and three-dimensional culture. In the case of two-dimensional culture, the pancreatic progenitor cells obtained in step 4) are treated with 0.25% trypsin-EDTA and then dispersed by pipetting, and the obtained dispersion is subjected to centrifugal separation. Recovered cells are resuspended in a fresh medium of step 5) and the cell suspension is reseeded to a fresh two-dimensional culture container. The culture period is 2 days to 3 days, preferably about 2 days.

The basal medium for use in the culture of mammalian cells described in step 1) can be used as culture medium. The medium is supplemented with SANT1, retinoic acid, ALK5 inhibitor II, T3, and LDN according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. The medium may be supplemented with dimethyl sulfoxide.

The culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

The pancreatic endocrine progenitor cells obtained in step 5) can be further purified using a known surface marker glycoprotein 2 (GP2) or the like. The purification can be performed by a method known per se, for example, using anti-GP2 antibody-immobilized beads.

### Step 6) Differentiation into insulin-producing cells

The pancreatic endocrine progenitor cells obtained in step 5) are further cultured in a medium containing a FGFR1 inhibitor to induce their differentiation into insulin-producing cells. The culture period is 10 days to 30 days, preferably about 10 to 20 days.

The basal medium for use in the culture of mammalian cells described in step 1) can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid according to the previous report (Nature Biotechnology 2014; 32: 1121-1133) and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428 .

The culture may be performed by any of two-dimensional culture and three-dimensional culture. Preferably, the culture is performed by nonadherent culture without the use of feeder cells. For the culture, a dish, a flask, a microplate, a porous plate (Nunc), or the like, or a bioreactor is used. The culture container is preferably surface-treated in order to decrease adhesiveness to cells.

The culture temperature is not particularly limited, and the culture is performed at 30 to 40°C (for example, 37°C). The concentration of carbon dioxide in a culture container is on the order of, for example, 5%.

The medium can contain a FGFR1 inhibitor in any amount capable of inhibiting FGFR1 activity and can contain the FGFR1 inhibitor in an amount of, for example, 10 µM or less or 5 µM or less, preferably in an amount of less than 5 µM, less than 4 µM, less than 3 µM, or less than 2 µM. The lower limit of the amount of the FGFR1 inhibitor added is not particularly limited and can be 0.1 µM or more, preferably 0.5 µM or more. The amount of the FGFR1 inhibitor added is preferably less than 5 µM and 0.1 µM or more, more preferably less than 5 µM and 0.5 µM or more. The culture in the presence of the FGFR1 inhibitor can be performed for at least 12 hours, preferably 24 hours or longer, 2 days or longer, 4 days or longer, 8 days or longer, 10 days or longer, or 15 days or longer. The culture in the presence of the FGFR1 inhibitor is preferably performed for 4 days or longer. The culture in the presence of the FGFR1 inhibitor can be performed, for example, for about last 4 to 15 days, preferably about last 4 to 7 days, of step 6). The medium may be replaced during the period of treatment with the FGFR1 inhibitor and can be replaced with a medium supplemented with the FGFR1 inhibitor, having the same or different composition as or from that before the replacement, according to the culture schedule.

The culture of the cells in the medium containing the FGFR1 inhibitor can suppress the proliferation of Ki67-positive cells in the resulting insulin-producing cells.

The insulin-producing cells obtained in step 6) can be dissociated and recovered using an enzyme such as trypsin. The recovered insulin-producing cells can be cryopreserved until use. Subsequently, the recovered insulin-producing cells can be seeded in an amount of about 500000 to 5000000 cells, preferably about 1000000 to 4000000 cells, more preferably about 2000000 to 3000000 cells, per incubator or well into the medium and subjected to three-dimensional culture to obtain insulin-producing cells in the form of spheroids. Each spheroid consists of about 100 to about 1000 cells, preferably about 200 to about 800 cells, more preferably about 300 to about 500 cells.

"Compound having tubulin polymerization-promoting activity and/or tubulin depolymerization-inhibiting activity" or "microtubule inhibitor" used in the present invention means a compound having activity of promoting the polymerization of tubulin that forms microtubule to excessively form or stabilize the microtubule, and/or activity of suppressing the depolymerization of tubulin that forms microtubule. The compound preferably has an effect of inhibiting cell division owing to the activity. As used herein, the term "compound having tubulin polymerization-promoting activity and/or tubulin depolymerization-inhibiting activity" is used interchangeably with "microtubule inhibitor".

"Compound having tubulin polymerization-promoting activity and/or tubulin depolymerization-inhibiting activity" or "microtubule inhibitor" that can be used in the present invention is not particularly limited as long as it has the activity. Examples thereof include compounds given below and pharmacologically acceptable salts and solvates thereof. These compounds may have one or more substituents or their substructures (substituents, rings, etc.) may be partially converted as long as the compounds have tubulin polymerization-promoting activity and/or tubulin depolymerization-inhibiting activity. Examples of the solvates include, but are not limited to, hydrates and acetone adducts.

**[Table 1]**

| Compound | General name: chemical name | Structure |
|---|---|---|
| 1 | Docetaxel: | 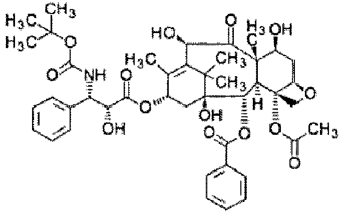 |
| | (-)-(1S,2S,3R,4S,5R,7S,8S,10R,13S)-4-Acetoxy-2-benzoyloxy-5,20-epoxy-1,7,10-trihydroxy-9-oxotax-11-en-13-yl (2R,3S)-3-tert-butoxycarbonylamido-2-hydroxy-3-phenylpropionate | |
| 2 | Paclitaxel: | 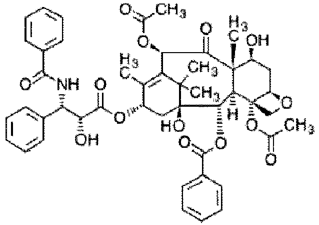 |
| | (-)-(1S,2S,3R,4S,5R,7S,8S,10R,13S)-4,10-Diacetoxy-2-benzoyloxy-5,20-epoxy-1,7-dihydroxy-9-oxotax-11-en-13-yl (2R,3S)-3-benzoylamino-2-hydroxy-3-phenylpropionate | |
| 3 | Cabazitaxel: | 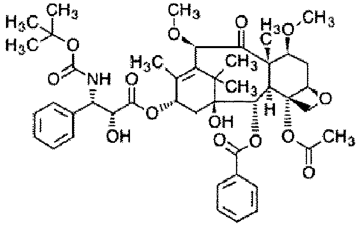 |
| | (1S,2S,3R,4S,5R,7S,8S,10R,13S)-4-Acetoxy-2-benzoyloxy-5,20-epoxy-1-hydroxy-7,10-dimethoxy-9-oxotax-11-ene-13-yl (2R,3S)-3-(1,1-dimethylethyl)oxycarbonylamino-2-hydroxy-3-phenylpropionate | |

In the present invention, "compound having tubulin polymerization-promoting activity and/or tubulin depolymerization-inhibiting activity" or "microtubule inhibitor" is preferably a compound that is used as an active ingredient for taxoid anticancer agents or a pharmacologically acceptable salt or a solvate thereof, more preferably docetaxel or a pharmacologically acceptable salt or a solvate thereof.

"Compound having tubulin polymerization-promoting activity and/or tubulin depolymerization-inhibiting activity" or "microtubule inhibitor" described above is commercially available or can be synthesized for use according to a known method.

In the present invention, the pancreatic endocrine progenitor cell population and/or the cell population at a later stage of differentiation, obtained by the induction of differentiation from pluripotent stem cells can be treated with the microtubule inhibitor by the contact of the cell population with the microtubule inhibitor. For example, the treatment with the microtubule inhibitor can be performed by culturing the cell population in a medium supplemented with the microtubule inhibitor. The microtubule inhibitor can be contained in any amount capable of reducing endogenous non-endocrine cells in the finally obtained insulin-producing cell population in the medium, and can be contained in an amount of, for example, 10 µM, 5 µM, 4 µM, 3 µM, 2 µM or less. The lower limit of the amount of the microtubule inhibitor added to the medium is not particularly limited and can be 0.1 µM, 0.2 µM, 0.3 µM, 0.4 µM, 0.5 µM, 1 µM or more. The range of the amount of the microtubule inhibitor added to the medium can be expressed using two numeric values respectively selected from the numeric values of the upper limit and the lower limit, and is, for example, 0.1 µM to 10 µM, preferably 0.1 µM to 5 µM, more preferably 1 µM to 3 µM.

The culture of the pancreatic endocrine progenitor cell population and/or the cell population at a later stage of differentiation in the presence of the microtubule inhibitor can be performed for any period capable of reducing endogenous non-endocrine cells in the finally obtained insulin-producing cell population. The culture can be performed for, for example, at least 12 hours, preferably 24 hours, 2 days, 3 days, 4 days, 5 days, 6 days, 7 days, 8 days, 10 days, 11 days, 12 days, 15 days or longer. The culture period of the pancreatic endocrine progenitor cell population and/or the cell population at a later stage of differentiation in the presence of the microtubule inhibitor is preferably 3 days, 4 days, 5 days, 6 days, 7 days, 8 days or longer. The medium may be replaced during the period of treatment with the microtubule inhibitor and can be replaced with a medium supplemented with the microtubule inhibitor, having the same or different composition as or from that before the replacement, according to the culture schedule. The basal medium for use in the culture of mammalian cells or the medium for induction of differentiation into insulin-producing cells can be used as culture medium.

In the present invention, the pancreatic endocrine progenitor cell population or the cell population at a later stage of differentiation, obtained by the induction of differentiation from pluripotent stem cells can be subjected to the step of differentiation into insulin-producing cells, in addition to being treated with the microtubule inhibitor. As used herein, "in addition to being treated with the microtubule inhibitor" includes the case of performing the step of treatment with the microtubule inhibitor and the step of differentiation at the same time, the case of treating the cell population with the microtubule inhibitor, followed by the step of differentiation, and the case of subjecting the cell population to the step of differentiation, followed by the step of treatment with the microtubule inhibitor. Thus, the medium for use in the treatment with the microtubule inhibitor and the medium for use in the differentiation of the cell population may be separate media, or the medium for use in the step of differentiation may be further supplemented with the microtubule inhibitor.

In one embodiment of the present invention, the microtubule inhibitor is contained in a medium in step 6 described above, and allowed to act on the cells, in the process of inducing the differentiation of pluripotent stem cells into insulin-producing cells. Preferably, the microtubule inhibitor is contained in the medium in step 6 over a period of 3 days, 4 days, 5 days, 6 days, 7 days, 8 days or longer before the completion of step 6 and allowed to act on the cells.

In another embodiment of the present invention, the microtubule inhibitor is contained in a medium and allowed to act on the cells after the completion of step 6 described above, in the process of inducing the differentiation of pluripotent stem cells into insulin-producing cells. The basal medium for use in the culture of mammalian cells described in step 1) can be used as culture medium. The medium is supplemented with ALK5 inhibitor II, T3, LDN, γ-secretase inhibitor XX, γ-secretase inhibitor RO, N-cysteine, an AXL inhibitor, and ascorbic acid and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428. Preferably, the microtubule inhibitor is contained in the medium over a period of 3 days, 4 days, 5 days, 6 days, 7 days, 8 days or longer after the completion of step 6 and allowed to act on the cells.

The proportion of insulin-producing cells, preferably insulin-positive and NKX6.1-positive cells, in the insulin-producing cell population obtained by the present invention is higher by 5% or more, preferably 10% or more, than the proportion of these cells in an insulin-producing cell population prepared by a conventional method (for example, steps 1) to 6) described above and Nature Biotechnology 2014; 32: 1121-1133). More specifically, the proportion of insulin-producing cells, more specifically, insulin-positive and NKX6.1-positive cells, in the insulin-producing cell population obtained by the present invention is 35% or more, preferably 36% or more, more preferably 37% or more, further preferably 38% or more, still further preferably 39% or more, particularly preferably 40% or more, especially preferably 41% or more. The upper limit of the proportion is not particularly limited and is 70% or less, 60% or less, or 50% or less. The proportion can be expressed using two numeric values respectively selected from the numeric values of the upper limit and the lower limit. The proportion is, for example, 35% to 50%, preferably 36% to 50%, more preferably 37% to 50%, further preferably 38% to 50%, still further preferably 39% to 50%, particularly preferably 40% to 50%, especially preferably 41% to 50%.

The proportion of chromogranin A (CHGA)-positive endocrine cells in the insulin-producing cell population obtained by the present invention is higher by 3% or more, preferably 4% or more, more preferably 5% or more, than the proportion of these cells in an insulin-producing cell population prepared by a conventional method (for example, steps 1) to 6) described above and Nature Biotechnology 2014; 32: 1121-1133). More specifically, the proportion of chromogranin A (CHGA)-positive, for example, CHGA-positive and PDX1-positive endocrine cells in the insulin-producing cell population obtained by the present invention is 95% or more, preferably 96% or more, more preferably 97% or more, further preferably 98% or more. The upper limit of the proportion is not particularly limited and is 99% or less. The proportion can be expressed using two numeric values respectively selected from the numeric values of the upper limit and the lower limit. The proportion is, for example, 95% to 99%, preferably 96% to 99%, more preferably 97% to 99%, further preferably 98% to 99%.

The insulin-producing cell population obtained by the present invention may include other cells (for example, pancreatic endocrine progenitor cells; other pancreatic hormone-producing cells expressing at least one of the markers glucagon, somatostatin, and pancreatic polypeptide; Ki67-positive cells and CHGA-negative cells), in addition to the insulin-producing cells and the endocrine cells.

The insulin-producing cell population obtained by the method of the present invention is an insulin-producing cell population in which endogenous non-endocrine cells have been reduced, preferably removed, as compared with an insulin-producing cell population prepared by a conventional method (for example, steps 1) to 6) described above and Nature Biotechnology 2014; 32: 1121-1133). As used herein, "non-endocrine cells" are cells characterized by a chromogranin A (CHGA)-negative marker expression pattern. The non-endocrine cells can be further characterized by being PDX1-negative and/or Ki67-positive. Preferably, "non-endocrine cells" are CHGA-negative and PDX1-positive cells, CHGA-negative and PDX1-negative cells, and/or CHGA-negative and Ki67-positive cells.

The proportion of non-endocrine cells in the insulin-producing cell population obtained by the present invention is lower by 2% or more, 3% or more, 4% or more, or 5% or more, than the proportion of these cells in an insulin-producing cell population prepared by a conventional method (for example, steps 1) to 6) described above and Nature Biotechnology 2014; 32: 1121-1133). More specifically, the proportion of non-endocrine cells in the insulin-producing cell population obtained by the present invention is 5% or less, preferably 4% or less, more preferably 3% or less, further preferably 2% or less, still further preferably 1% or less, particularly preferably 0.5% or less. The lower limit of the proportion is not particularly limited and is, for example, 0% or more, 0.01% or more, or 0.05% or more. The proportion can be expressed using two numeric values respectively selected from the numeric values of the upper limit and the lower limit. The proportion is, for example, 0% to 5%, preferably 0.01% to 4%, more preferably 0.01% to 3%, further preferably 0.01% to 2%, still further preferably 0.01% to 1%, particularly preferably 0.01% to 0.5%. More specifically, the proportion of CHGA-negative and Ki67-positive cells in the insulin-producing cell population obtained by the present invention is 0.1% or less, preferably 0.05% or less. The lower limit of the proportion is not particularly limited and is, for example, 0% or more or 0.01% or more. The proportion can be expressed using two numeric values respectively selected from the numeric values of the upper limit and the lower limit. The proportion is, for example, 0% to 0.1% or 0.01% to 0.1%.

The present invention also provides a method for detecting non-endocrine cells present in an insulin-producing cell population obtained by the induction of differentiation from pluripotent stem cells.

In the present invention, the non-endocrine cells present in the insulin-producing cell population are allowed to proliferate by subjecting the insulin-producing cell population to further culture (also referred to as "extended culture" herein), and can thereby be easily detected. The extended culture can be performed using the basal medium for use in the culture of mammalian cells, and MCDB131/20 mM Glucose/NaHCO₃/FAF-BSA/ITS-X/Glutamax/Penicillin Streptomycin medium is preferably used as the basal medium.

Preferably, the medium for the extended culture further contains epidermal growth factor (EGF) or a substance having equivalent or similar activity thereto. Examples of "substance having equivalent or similar activity" to EGF include, but are not limited to, substances capable of activating an EGF signal transduction pathway (for example, betacellulin), and growth factors functionally similar to EGF (for example, fibroblast growth factor (FGF), platelet-derived growth factor (PDGF), and vascular endothelial growth factor (VEGF)). The EGF or the substance having equivalent or similar activity thereto can be contained in any amount sufficient for the proliferation of the non-endocrine cells in the medium. For example, the EGF or the substance having equivalent or similar activity thereto can be contained in an amount selected from the range of 50 ng/mL, 60 ng/mL, 70 ng/mL, 80 ng/mL, 90 ng/mL or more in the medium. The upper limit thereof is not particularly limited and can be an amount selected from the range of 250 ng/mL, 200 ng/mL, 150 ng/mL, 100 ng/mL or less. The content can be expressed using two numeric values respectively selected from the numeric values of the upper limit and the lower limit. The content is, for example, an amount selected from the range of 50 ng/mL to 250 ng/mL, 50 ng/mL to 200 ng/mL, 50 ng/mL to 150 ng/mL, or 50 ng/mL to 100 ng/mL.

The period of the extended culture can be a period sufficient for the proliferation of the non-endocrine cells and is at least 12 hours, preferably 24 hours or longer, for example, 2 days, 3 days, 4 days, 5 days, 7 days, 10 days, 14 days, 21 days, 27 days, 28 days, 29 days, 30 days or longer. The medium may also be replaced during the period of the extended culture and can be replaced with a fresh one at a frequency of every 1 to 7 days, preferably every 2 to 5 days, more preferably every 3 to 4 days.

In the case of subjecting the insulin-producing cell population obtained by the present invention to the extended culture, the proportion of non-endocrine cells in the population confirmed after the extended culture is reduced by 50% or more, preferably 65% or more, more preferably 70% or more, further preferably 75% or more, as compared with the proportion of these cells in the case of subjecting an insulin-producing cell population prepared by a conventional method (for example, steps 1) to 6) described above and Nature Biotechnology 2014; 32: 1121-1133) to the extended culture. Thus, it can be confirmed that endogenous non-endocrine cells have been reduced, preferably removed, in the insulin-producing cell population obtained by the present invention.

The differentiation of the insulin-producing cell population obtained by the present invention into a cell population comprising pancreatic β cells (hereinafter, referred to as "pancreatic β cell population") can be induced. As used herein, "pancreatic β cells" means cells more mature than "insulin-producing cells" and specifically means cells characterized by expressing at least one of the markers MAFA, UCN3, and IAPP, which are maturation markers of pancreatic β cells, or by a reaction to increase insulin secretion by glucose stimulation. The pancreatic β cell population may include other cells (for example, insulin-producing cells, Ki67-positive cells and CHGA-negative cells), in addition to the pancreatic β cells.

The pancreatic β cell population can be obtained by the differentiation and/or maturation, preferably the *in vivo* differentiation and/or maturation in an animal, of the insulin-producing cell population.

"Animal" is preferably a mammal. Examples thereof include humans, nonhuman primates, pigs, cattle, horses, sheep, goats, llamas, dogs, cats, rabbits, mice, and guinea pigs. A human is preferred.

The transplantation is preferably performed to an *in vivo* region where the cell population can be fixed at a given position, and can be performed, for example, subcutaneously, intraperitoneally, to the peritoneal mesothelium, to the greater omentum, to a fat tissue, to a muscle tissue, or beneath the capsule of each organ such as the pancreas or the kidney, in the animal. The number of cells to be transplanted may vary depending on factors such as the stage of differentiation of the cells to be transplanted, the age and body weight of a recipient, the size of a transplantation site in a recipient, and the severity of a disease of a recipient and is not particularly limited. For example, the number of cells can be on the order of 10 × 10⁴ cells to 10 × 10¹¹ cells. The transplanted cell population is induced to differentiate in an *in vivo* environment and can thereby differentiate into the cell population of interest, preferably a pancreatic β cell population, which may then be recovered or may be indwelled *in vivo* as it is.

For the transplantation, the cell population may be embedded in a gel containing a biocompatible material and then transplanted. For example, the cell population embedded in the gel containing a biocompatible material may be enclosed in a device such as a capsule, a bag, or a chamber and transplanted into a living body.

As used herein, "embedding" means that an pancreatic endocrine progenitor cell population or a cell population at a later stage of differentiation is contained in a scattered manner in the gel containing a biocompatible material.

As used herein, "biocompatible material" means an arbitrary material that induces neither marked immune response nor harmful biological reaction (for example, toxic reaction and blood coagulation) when transplanted into a living body and indwelled for a short period or a long period. Also, "biocompatible material" is preferably a biodegradable material. Examples of such a material include polylactic acid (PLA), polycaprolactone (PCL), polyurethane (PU), polyethylene glycol (PEG), polyhydroxyethyl methacrylate, polyglycolic acid (PGA), poly(lactic-co-glycolic acid) (PLGA), poly(3-hydroxybutyrate-co-hydroxyvalerate) (PHBV), poly(ethylene-co-vinyl acetate) (PEVA), polyacrylamide, polyethylene oxide, polyethyleneamine, polyhydroxybutyric acid, poly(N-vinylpyrrolidone), polyvinyl alcohol, polypropylene fumarate, polyacrylic acid, poly-e-caprolactone, polymethacrylic acid, polyvinylidene difluoride (PVDF), pectic acid, hyaluronic acid, heparin sulfate, chondroitin sulfate, heparan sulfate proteoglycan, heparin, chitin, chitosan, xanthan, carboxymethylcellulose, carboxymethyl chitosan, alginate, alginic acid ester, collagen, cellulose, silk fibroin, keratin, gelatin, fibrin, pullulan, laminin, gellan, silicon, urethane, elastin and modified forms thereof, and combinations thereof. The surface of "biocompatible material" may be modified (for example, coated with a substrate for cell adhesion (collagen, gelatin, poly-L-lysine, poly-D-lysine, laminin, fibronectin, Matrigel, vitronectin, etc.)) so as to permit cell adhesion or may be engineered with a functional group (for example, an amino group, a carboxyl group, a hydroxy group, a methacrylic acid group, and an acrylic acid group) known to control cell proliferation, differentiation, or functions, if necessary. In a particular embodiment, alginate or alginic acid ester can be suitably used as "biocompatible material".

The alginate can be a water-soluble salt, and a metal salt, an ammonium salt, or the like can be used. For example, sodium alginate, calcium alginate, or ammonium alginate can be suitably used.

The alginic acid ester (also referred to as propylene glycol alginate) is a derivative in which propylene glycol is bonded to the carboxyl group of alginic acid through an ester bond.

The ratio of mannuronic acid to guluronic acid (M/G ratio) contained in the alginate is arbitrary. In general, in the case of M > G, a highly flexible gel can be formed. In the case of M < G, a strong gel can be formed. In the present invention, alginate containing guluronic acid at a proportion of 10 to 90%, 20 to 80%, 30 to 70%, or 40 to 60% can be used.

The gel can be prepared using alginate or alginic acid ester in accordance with a known approach (WO2010/032242 and WO2011/154941) and can be obtained by adding a cross-linking agent to a solution of alginate or alginic acid ester for gelation.

The alginate or the alginic acid ester can be contained in an amount of 0.05 to 10% by weight, preferably 0.1 to 5% by weight, more preferably 0.5 to 3% by weight, in a solvent. The solvent can be any solvent capable of dissolving the alginate or the alginic acid ester, and water, physiological saline, or the like can be used.

The cross-linking agent can be any cross-linking agent that can allow a solution of alginate or alginic acid ester to gelate, and is not particularly limited. A polyvalent metal cation can be used. The polyvalent metal cation is preferably a divalent metal cation, more preferably a calcium ion, a strontium ion, or a barium ion. The cross-linking agent can be used in the form of a salt. In the present invention, at least one member selected from calcium chloride, strontium chloride, and barium chloride can be used as the cross-linking agent.

The gel containing alginate or alginic acid ester can contain a nanofiber. The nanofiber is a natural or synthetic fiber having a diameter of a nanometer order. Examples of the natural nanofiber include nanofibers containing one or more of collagen, cellulose, silk fibroin, keratin, gelatin, and polysaccharides such as chitosan. Examples of the synthetic nanofiber include polylactic acid (PLA), polycaprolactone (PCL), polyurethane (PU), poly(lactide-co-glycolide) (PLGA), poly(3-hydroxybutyrate-co-hydroxyvalerate) (PHBV), and poly(ethylene-co-vinylacetate) (PEVA). The nanofiber can be contained in an amount of less than 1% by weight, for example, 0.9% by weight, 0.8% by weight, 0.7% by weight, 0.6% by weight, 0.5% by weight, or less than the amount, in the gel containing alginic acid. The lower limit of the amount of the nanofiber contained in the gel containing alginate or alginic acid ester is not particularly limited and can be 0.05% by weight or more, preferably 0.1% by weight or more.

The embedding of the cell population in the gel containing alginate or alginic acid ester can be performed by an arbitrary approach and can be performed, for example, by mixing the cell population into a solution of alginate or alginic acid ester and gelating it, though the embedding is not particularly limited thereto.

The cell population can be contained in an amount selected from 1 × 10⁴ cells to 1 × 10⁹ cells/mL, preferably 1 × 10⁷ cells to 1 × 10⁸ cells/mL, in the solution of alginate or alginic acid ester.

The gelation of the solution of alginate or alginic acid ester containing the cell population can be performed by adding a cross-linking agent to the solution. The amount of the cross-linking agent added can be an amount selected from 0.1 to 5% by weight, for example, 0.1 to 1% by weight, with respect to the solution. The gelation can be performed in a container having a predetermined configuration and/or shape for use in cell culture or cell transplantation, or in a mold designed so as to obtain a gel adapted to the container.

Alternatively, the gelation may be performed by forming a gel capsule containing alginic acid in accordance with a known approach (WO2010/010902). Specifically, the solution of alginate or alginic acid ester containing the cell population may be added dropwise to a solution of a cross-linking agent for gelation. The size of liquid droplets can be adjusted according to the shape of a nozzle for dropwise addition or a dropwise addition method, and by extension, the size of the gel capsule containing alginic acid can be defined. The dropwise addition method is not particularly limited and can be performed by an approach such as an air spray method, an airless spray method, or a static spray method. The size of the gel capsule containing alginic acid is not particularly limited and can be a diameter of 5 mm or smaller, 1 mm or smaller, or 500 µm or smaller. The cross-linking agent solution can contain the cross-linking agent in an amount selected from 0.1 to 10% by weight, for example, 0.1 to 5% by weight.

The insulin-producing cell population obtained by the present invention may be indwelled as it is and used as insulin-producing and/or -secreting cells, when transplanted into a living body of an animal and differentiated in the living body of the animal.

The insulin-producing cell population obtained by the present invention is transplanted as it is or in a capsule form to an affected area and is thereby useful as a cell medicine for treating diabetes mellitus, particularly, type I diabetes mellitus.

The insulin-producing cell population obtained by the present invention may be a prodrug. As used herein, the prodrug refers to a cell population that is differentiated after transplantation into a living body and converted to cells having a function of treating a disease.

The insulin-producing cell population obtained by the present invention has low toxicity (for example, acute toxicity, chronic toxicity, genetic toxicity, reproductive toxicity, cardiotoxicity, and carcinogenicity) and can be safely administered as it is or in the form of a pharmaceutical composition containing the cell population mixed with a pharmacologically acceptable carrier, etc. to a mammal (for example, a mouse, a rat, a hamster, a rabbit, a cat, a dog, cattle, sheep, a monkey, and a human).

### 3. Culture medium

The present invention provides a culture medium for a pancreatic endocrine progenitor cell population and/or a cell population at a later stage of differentiation, comprising a microtubule inhibitor.

The culture medium of the present invention is prepared by adding the microtubule inhibitor in the amount described above to a basal medium for use in the culture of mammalian cells, such as RPMI 1640 medium, MEM medium, iMEM medium, DMEM/F12 medium, Improved MEM Zinc Option medium, Improved MEM/1% B-27/Penicillin Streptomycin medium, or MCDB131/20 mM Glucose/NaHCO₃/FAF-BSA/ITS-X/GlutaMAX(TM)/ascorbic acid/Penicillin Streptomycin medium.

The culture medium of the present invention can be a differentiation medium for inducing the differentiation of a pancreatic endocrine progenitor cell population and/or a cell population at a later stage of differentiation into an insulin-producing cell population. The differentiation medium of the present invention can be used in step 6) in the aforementioned method for inducing the differentiation of pluripotent stem cells into insulin-positive cells.

The differentiation medium of the present invention further contains other factors required for step 6), such as a growth factor, various inhibitors, a serum replacement, an antibiotic, and a vitamin, in addition to the microtubule inhibitor. According to the previous report (Nature Biotechnology 2014; 32: 1121-1133), for example, the differentiation medium for use in step 6) can be supplemented with predetermined amounts of ALK5 inhibitor II, T3, LDN, γ-secretase inhibitor XX, γ-secretase inhibitor RO, N-cysteine, an AXL inhibitor, ascorbic acid, a Wnt inhibitor, a ROCK inhibitor, FGF (preferably FGF2), a serum replacement, a vitamin, an antibiotic, ZnSO₄, heparin, N-acetylcysteine, Trolox, R428, a FGFR1 inhibitor, and the like.

The culture medium of the present invention can be a culture medium for culturing "cell population at a later stage of differentiation" described above. In one embodiment, the culture medium can be used for a period of 3 days, 4 days, 5 days, 6 days, 7 days, 8 days or longer before the completion of step 6) or after the completion of step 6), in the aforementioned method for inducing the differentiation of pluripotent stem cells into insulin-positive cells. The culture medium is further supplemented with ALK5 inhibitor II, T3, LDN, γ-secretase inhibitor XX, γ-secretase inhibitor RO, N-cysteine, an AXL inhibitor, and ascorbic acid, in addition to the microtubule inhibitor, and may be appropriately further supplemented with a Wnt inhibitor, a ROCK inhibitor, FGF (preferably FGF2), a serum replacement, a vitamin, an antibiotic, and the like. For example, the medium may be supplemented with ALK5 inhibitor II, T3, LDN, γ-secretase inhibitor RO, and ascorbic acid or may be supplemented with T3, ALK5 inhibitor II, ZnSO₄, heparin, N-acetylcysteine, Trolox, and R428.

The present invention also provides a culture medium for an insulin-producing cell population, comprising EGF or a substance having equivalent or similar activity thereto.

The culture medium of the present invention comprises the EGF or the substance having equivalent or similar activity thereto in any amount capable of promoting the proliferation of non-endocrine cells in the basal medium. The basal medium is preferably MCDB131/20 mM Glucose/NaHCO₃/FAF-BSA/ITS-X/Glutamax/Penicillin Streptomycin medium. The amount of the EGF or the substance having equivalent or similar activity thereto, contained in the medium is as described above.

The culture medium comprising the EGF or the substance having equivalent or similar activity thereto is a culture medium for detecting non-endocrine cells present in an insulin-producing cell population and can be used in the aforementioned extended culture of the insulin-producing cell population.

Every culture medium of the present invention may be provided in one form of a mixture of a basal medium, the microtubule inhibitor or the EGF or the substance having equivalent or similar activity thereto, and other factors described above, or may be provided in any combination or in a plurality of separate forms of these components and prepared just before use.

### 4. Method for removing non-endocrine cells

The present invention also relates to a method for removing non-endocrine cells, comprising treating a pancreatic endocrine progenitor cell population obtained by the induction of differentiation from pluripotent stem cells, and/or a cell population at a later stage of differentiation with a microtubule inhibitor.

The treatment of the pancreatic endocrine progenitor cell population and/or the cell population at a later stage of differentiation, obtained by the induction of differentiation from pluripotent stem cells with the microtubule inhibitor can be performed as defined above.

Hereinafter, the present invention will be described with reference to Examples. However, the present invention is not limited by these Examples.

### Examples

### Example 1: Proliferation evaluation of non-endocrine cells in extended culture of insulin-producing cell population in basal medium containing EGF

### 1. Method

### (1) Preparation of insulin-producing cell population

The differentiation of a Ff-I14s04 iPS cell line into an insulin-producing cell population was induced by the three-dimensional culture method using a bioreactor and a multiwell plate according to steps 1) to 6) described above and the previous report (Nature Biotechnology 2014; 32: 1121-1133). Specifically, the iPS cells were subjected to the first culture in a medium (DMEM/1% B-27(INS+)/Penicillin Streptomycin/dimethyl sulfoxide) containing differentiation-inducing factors (GSK3β inhibitor, ROCK inhibitor and low dose of activin A) under conditions causing action of insulin, and subsequently subjected to the second culture in a medium (DMEM/1% B-27(INS-)/Penicillin Streptomycin/dimethyl sulfoxide) containing a differentiation-inducing factor (low dose of activin A) under conditions causing no action of insulin to obtain a definitive endoderm cell population. Further, a pancreatic endocrine progenitor cell population obtained by the induction of differentiation from the definitive endoderm cell population was cultured in a medium (Improved MEM/1% B-27(INS+)/Penicillin Streptomycin) containing differentiation-inducing factors (ALK5 inhibitor II, T3, LDN, γ-secretase inhibitor RO, and ascorbic acid) and FGF receptor 1 inhibitor (PD-166866) to obtain an insulin-producing cell population. The exchange from the bioreactor to the multiwell plate was carried out in step 6).

### (2) Extended culture using basal medium containing EGF

The insulin-producing cell population was subjected to extended culture for 4 weeks in a basal medium (MCDB131/20 mM Glucose/NaHCO₃/FAF-BSA/ITS-X/Glutamax/Penicillin Streptomycin). EGF, when added for the purpose of promoting the proliferation of unintended cells, was added at 50 ng/mL (final concentration) to the basal medium for culture.

### (3) Evaluation of marker protein expression and the number of cells

Marker protein expression (PDX1 and CHGA) in the insulin-producing cell population before the start of and after the completion of extended culture was measured by flow cytometry. Respective samples were collected by sampling from separate culture containers. The number of cells was measured in an automatic cell counter at the time of sample preparation for flow cytometry, and the total number of cells per well of the multiwell plate was calculated. The number of CHGA-positive cells, the number of PDX1-positive and CHGA-negative cells, and the number of PDX1-negative and CHGA-negative cells were calculated from the obtained total number of cells and the flow cytometry results.

### 2. Results

### (1) Evaluation of marker protein expression and the number of cells

Figure 1 and Table 2 show results of measurement by flow cytometry and the number of cells. Most cells before extended culture were CHGA-positive endocrine cells. Most cells remained CHGA-positive cells even when extended culture for 4 weeks was performed in a basal medium containing no EGF. However, extended culture in a basal medium containing EGF markedly increased the proportions of PDX1-positive and CHGA-negative (PDX1+/CHGA-) and PDX1-negative and CHGA-negative (PDX1-/CHGA-) non-endocrine cells. Furthermore, these non-endocrine cells were confirmed to be increased not only in proportion but in the number of cells. The total number of cells after extended culture in the basal medium containing EGF was also increased with increase in the number of non-endocrine cells. On the other hand, there was no large change in the number of CHGA-positive endocrine cells.

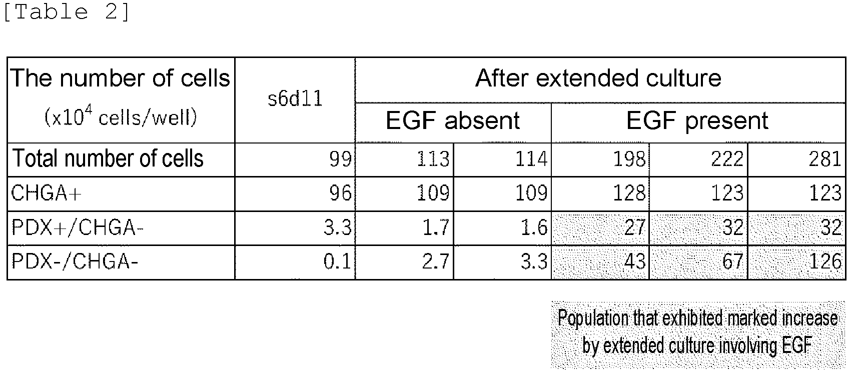

### Example 2: Proliferation evaluation of non-endocrine cells in extended culture of insulin-producing cell population prepared by addition of taxoid anticancer agent docetaxel in basal medium containing EGF

### 1. Method

### (1) Preparation of insulin-producing cell population by induction step involving cisplatin or docetaxel treatment

The differentiation of a QHJI-I14s04 iPS cell line into an insulin-producing cell population was induced by the three-dimensional culture method using a bioreactor according to steps 1) to 6) described above and the previous report (Nature Biotechnology 2014; 32: 1121-1133). Specifically, the iPS cells were subjected to the first culture in a medium (DMEM/1% B-27(INS+)/Penicillin Streptomycin/dimethyl sulfoxide) containing differentiation-inducing factors (GSK3β inhibitor, ROCK inhibitor and low dose of activin A) under conditions causing action of insulin, and subsequently subjected to the second culture in a medium (DMEM/1% B-27(INS-)/Penicillin Streptomycin/dimethyl sulfoxide) containing a differentiation-inducing factor (low dose of activin A) under conditions causing no action of insulin to obtain a definitive endoderm cell population. Further, a pancreatic endocrine progenitor cell population obtained by the induction of differentiation from the definitive endoderm cell population was cultured in a medium (Improved MEM/1% B-27(INS+)/Penicillin Streptomycin) containing differentiation-inducing factors (ALK5 inhibitor II, T3, LDN, γ-secretase inhibitor RO, and ascorbic acid) and FGF receptor 1 inhibitor (PD-166866) to obtain an insulin-producing cell population. The cells, when treated with cisplatin or docetaxel, were induced in the medium supplemented with 3 µM (final concentration) cisplatin or 1 µM (final concentration) docetaxel for 7 days from 4 days after the start of step 6) to the completion of culture (day 11).

### (2) Extended culture using basal medium containing EGF

The insulin-producing cell population obtained by the addition of cisplatin or docetaxel was subjected to extended culture for 4 weeks in a basal medium (MCDB131/20 mM Glucose/NaHCO₃/FAF-BSA/ITS-X/Glutamax/Penicillin Streptomycin) containing 50 ng/mL (final concentration) EGF. An insulin-producing cell population obtained without the addition of cisplatin and docetaxel was used as a control cell population and subjected to extended culture in the same manner as above.

### (3) Evaluation of marker protein expression and the number of cells

Marker protein expression (PDX1, CHGA, and Ki67) in each insulin-producing cell population before the start of and after the completion of extended culture was measured by flow cytometry. Samples after the completion of extended culture were collected by sampling three times from the same culture container. The number of cells was measured in an automatic cell counter at the time of sample preparation for flow cytometry, and the total number of cells per mL of the bioreactor was calculated. The number of CHGA-positive cells, the number of PDX1-positive and CHGA-negative cells, and the number of PDX1-negative and CHGA-negative cells were calculated from the obtained total number of cells and the flow cytometry results.

### 2. Results

### (1) Evaluation of marker protein expression and the number of cells

Figures 2 and 3 and Tables 3, 4, and 5 show results of measurement by flow cytometry and the number of cells. In a control cell population, most cells before extended culture were CHGA-positive endocrine cells. Extended culture in a basal medium containing EGF markedly increased the proportions of PDX1-positive and CHGA-negative (PDX1+/CHGA-) and PDX1-negative/CHGA-negative (PDX1-/CHGA-) non-endocrine cells.

A cisplatin-supplemented cell population (+Cisplatin) exhibited no large change in the proportion of PDX1-positive and CHGA-negative (PDX1+/CHGA-) cells after extended culture involving EGF with respect to the control cell population (Control), whereas the proportion of PDX1-negative and CHGA-negative (PDX1-/CHGA-) cells was drastically decreased.

In a docetaxel-supplemented cell population (+Docetaxel), both the proportions of PDX1-positive and CHGA-negative (PDX1+/CHGA-) and PDX1-negative and CHGA-negative (PDX1-/CHGA-) cells after extended culture involving EGF were drastically decreased with respect to the control cell population.

The total number of cells after extended culture involving EGF was increased with increase in the number of non-endocrine cells in the control cell population and the cisplatin-supplemented cell population. The total number of cells after extended culture was decreased in the docetaxel-supplemented cell population because the number of non-endocrine cells was not increased. On the other hand, the number of CHGA-positive endocrine cells after extended culture was not changed in the cisplatin-supplemented cell population or the docetaxel-supplemented cell population with respect to the control cell population. These results suggest that cisplatin or docetaxel treatment suppresses increase in the number of non-endocrine cells and on the other hand, does not influence the number of endocrine cells.

The PDX1-positive and CHGA-negative and PDX1-negative and CHGA-negative non-endocrine cells increased in number by extended culture contained many cells positive to the proliferation marker Ki67. Thus, these non-endocrine cells are considered proliferative.

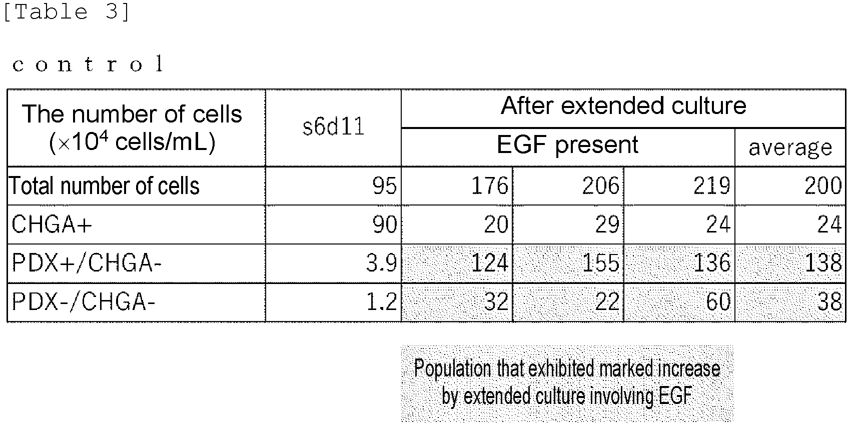

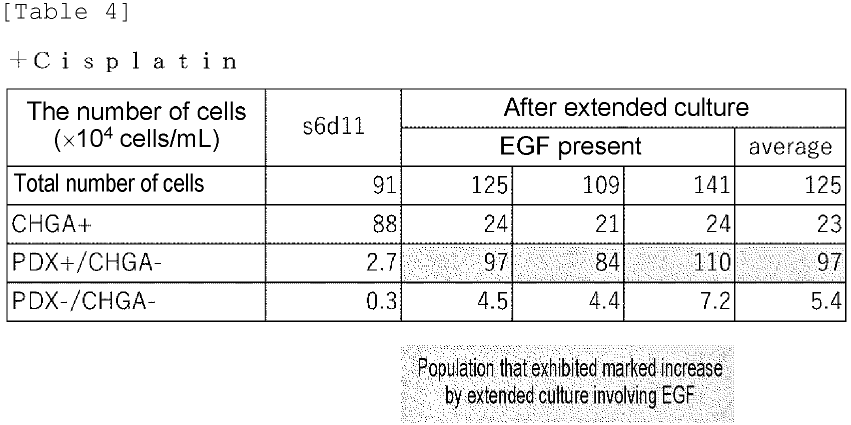

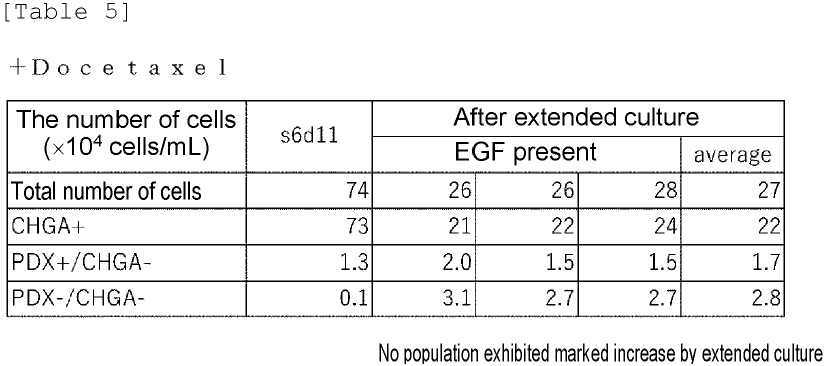

### Example 3: Marker protein expression evaluation of insulin-producing cell population prepared by addition of docetaxel

### 1. Method

### (1) Preparation of insulin-producing cell population by induction step involving docetaxel treatment

The differentiation of a QHJI-I14s04 iPS cell line into insulin-producing cells was induced by the three-dimensional culture method using a bioreactor and a multiwell plate according to steps 1) to 6) described above and the previous report (Nature Biotechnology 2014; 32: 1121-1133).
Specifically, the iPS cells were subjected to the first culture in a medium (DMEM/1% B-27(INS+)/Penicillin Streptomycin/dimethyl sulfoxide) containing differentiation-inducing factors (GSK3β inhibitor, ROCK inhibitor and low dose of activin A) under conditions causing action of insulin, and subsequently subjected to the second culture in a medium (DMEM/1% B-27(INS-)/Penicillin Streptomycin/dimethyl sulfoxide) containing a differentiation-inducing factor (low dose of activin A) under conditions causing no action of insulin to obtain definitive endoderm cells. Further, a pancreatic endocrine progenitor cell population obtained by the induction of differentiation from the definitive endoderm cells was cultured in a medium (MCDB131/20 mM Glucose/NaHCO₃/FAF-BSA/ITS-X/Glutamax/Penicillin Streptomycin) containing differentiation-inducing factors (ALK5 inhibitor II, T3, LDN, γ-secretase inhibitor RO, and ascorbic acid) and FGF receptor 1 inhibitor (PD-166866) to obtain an insulin-producing cell population. The exchange from the bioreactor to the multiwell plate was carried out in step 6). The cells, when treated with docetaxel, were induced in the medium supplemented with 1, 3 or 10 µM (final concentration) docetaxel for 7 days from 4 days after the start of step 6) to the completion of culture (day 11).

### (2) Evaluation of marker protein expression and the number of cells

Marker protein expression (NKX6.1, C-peptide (insulin), CHGA, and Ki67) in each insulin-producing cell population after the completion of culture was measured by flow cytometry. Respective samples were collected by sampling from separate culture containers. The number of cells was measured in an automatic cell counter at the time of sample preparation for flow cytometry, and the total number of cells per well of the multiwell plate was calculated.

### 2. Results

### (1) Evaluation of marker protein expression and the number of cells

Figure 4 shows results of measurement by flow cytometry and the number of cells. The proportion of INS-positive and NKX6.1-positive cells from which maturation into pancreatic β cells was predicted was increased by about 10% in a cell population treated with 1 µM docetaxel (+Docetaxel) with respect to a control cell population. This action was not changed up to the docetaxel concentration of 10 µM. The total number of cells was not changed by docetaxel treatment.

In addition, the abundance of CHGA-negative and Ki67-positive (CHGA-/Ki67+) cells which are unintended cells are drastically reduced in a cell population obtained by the induction of differentiation using a medium supplemented with FGF receptor 1 inhibitor (PD-166866) (see WO2019/208505). Thus, the abundance thereof was 0.1% or less, irrespective of the presence or absence of docetaxel treatment. In the case of performing evaluation without extended culture, as in this Example, the difference of slightly remaining unintended cells may be difficult to detect. On the other hand, extended culture in a basal medium containing EGF as described above enables slightly remaining unintended cells to proliferate and be detected, and thus enables the type or amount of cells contained in a cell population to be precisely understood.

### Example 4: Proliferation evaluation of non-endocrine cells in docetaxel treatment followed by extended culture of cell population comprising insulin-producing cells in basal medium containing EGF

### 1. Method

### (1) Preparation of insulin-producing cell population by docetaxel treatment of cell population comprising insulin-producing cells

The differentiation of a QHJI-I14s04 iPS cell line into an insulin-producing cell population was induced by the three-dimensional culture method using a bioreactor according to steps 1) to 6) described above and the previous report (Nature Biotechnology 2014; 32: 1121-1133). Specifically, the iPS cells were subjected to the first culture in a medium (DMEM/1% B-27(INS+)/Penicillin Streptomycin/dimethyl sulfoxide) containing differentiation-inducing factors (GSK3β inhibitor, ROCK inhibitor and low dose of activin A) under conditions causing action of insulin, and subsequently subjected to the second culture in a medium (DMEM/1% B-27(INS-)/Penicillin Streptomycin/dimethyl sulfoxide) containing a differentiation-inducing factor (low dose of activin A) under conditions causing no action of insulin to obtain a definitive endoderm cell population. Further, a pancreatic endocrine progenitor cell population obtained by the induction of differentiation from the definitive endoderm cell population was cultured for 7 days in a medium (Improved MEM/1% B-27(INS+)/Penicillin Streptomycin) containing differentiation-inducing factors (ALK5 inhibitor II, T3, LDN, γ-secretase inhibitor RO, and ascorbic acid) and FGF receptor 1 inhibitor (PD-166866) to obtain a cell population. The resulting cell population (obtained 7 days after the start of step 6) was subsequently induced in the medium supplemented with 1 µM (final concentration) docetaxel for 4 days to the completion of culture in step 6) (day 11). The exchange from the bioreactor to the multiwell plate was carried out in step 6) .

### (2) Extended culture using basal medium containing EGF

The cell population obtained by the approach described above (+Docetaxel 4 days from day 7) was subjected to extended culture for 4 weeks in a basal medium (MCDB131/20 mM Glucose/NaHCO₃/FAF-BSA/ITS-X/Glutamax/Penicillin Streptomycin) containing 50 ng/mL (final concentration) EGF. An insulin-producing cell population obtained without the addition of docetaxel (Control) or a cell population prepared in the same manner as above except that docetaxel treatment was performed for 7 days from 4 days after the start of step 6) to the completion of culture (day 11) (+Docetaxel 7 days from day 4) was subjected to extended culture in the same manner as above.

### (3) Evaluation of marker protein expression and the number of cells

Marker protein expression (PDX1 and CHGA) in each cell population before the start of and after the completion of extended culture was measured by flow cytometry. Marker protein expression (NKX6.1, C-peptide (insulin), PDX1, and CHGA) in the cell population obtained 7 days after the start of step 6) was also measured by flow cytometry. Respective samples were collected by sampling from separate culture containers. The number of cells was measured in an automatic cell counter at the time of sample preparation for flow cytometry, and the total number of cells per well of the multiwell plate was calculated.

### 2. Results

### (1) Evaluation of marker protein expression and the number of cells

Figure 5 and Tables 6, 7, and 8 show results of measurement by flow cytometry and the number of cells.

Figure 5(A) shows results of measuring marker protein expression in a cell population obtained 7 days after the start of step 6). The cell population was confirmed to be rich in cells positive to insulin, NKX6.1, PDX1, and CHGA (insulin-producing cells expressing both the markers insulin and NKX6.1 were present at a proportion of more than 30%; and cells expressing both the markers CHGA and PDX1 were present at a proportion of about 90%).

In a control cell population, most cells before extended culture were CHGA-positive endocrine cells. Extended culture in a basal medium containing EGF markedly increased the proportions of PDX1-positive and CHGA-negative (PDX1+/CHGA-) and PDX1-negative/CHGA-negative (PDX1-/CHGA-) non-endocrine cells.

In both of docetaxel-supplemented cell populations of "+Docetaxel 4 days from day 7" and "+Docetaxel 7 days from day 4", the proportions of PDX1-positive and CHGA-negative (PDX1+/CHGA-) and PDX1-negative and CHGA-negative (PDX1-/CHGA-) cells after extended culture involving EGF were drastically decreased with respect to the control cell population.

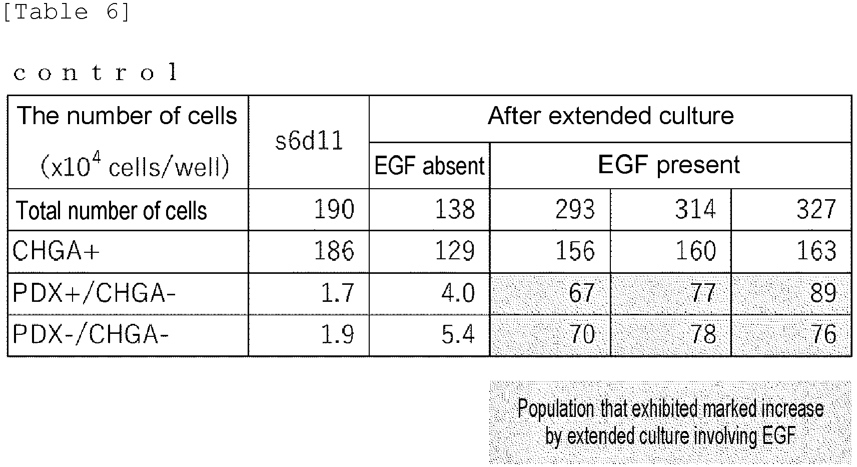

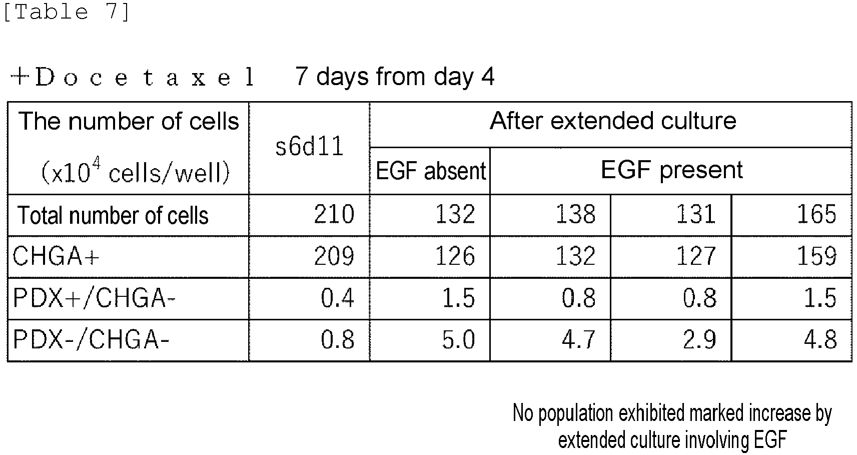

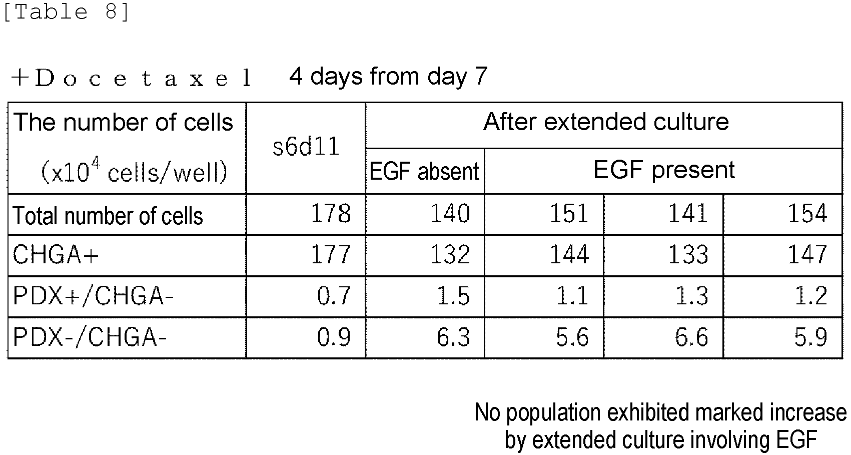

From these results, it was confirmed that coexisting proliferative non-endocrine cells in an insulin-producing cell population can be detected by the extended culture of the cell population in a basal medium containing EGF.

It was also revealed that in the process of inducing differentiation into an insulin-producing cell population, a pancreatic endocrine progenitor cell population and a cell population at a later stage of differentiation are treated with a taxoid anticancer agent such as docetaxel, whereby non-endocrine cells are reduced and an insulin-producing cell population comprising endocrine cells and insulin-producing cells at high proportions can be produced.

### Example 5: Efficacy and safety evaluation by in vivo transplantation of insulin-producing cell population prepared by addition of docetaxel

### 1. Method

### (1) In vivo transplantation of insulin-producing cell population prepared by addition of docetaxel

An immunodeficient NOD/SCID mouse was prepared by inducing insulin-deficient diabetes mellitus with streptozotocin. An insulin-producing cell population prepared by the addition of docetaxel by the method described in Example 2 was dispersed in a fibrin gel, and this dispersion was subcutaneously transplanted to the mouse. After transplantation, a human C-peptide concentration in plasma was measured over time. The graft was excised 6 months after transplantation, and its weight was measured. An insulin-producing cell population prepared without the addition of docetaxel was subcutaneously transplanted as a control to the mouse, and a human C-peptide concentration in plasma over time and the weight of the graft excised 6 months after transplantation were measured in the same manner as above.

### (2) Evaluation of non-endocrine cells in graft and protein expression

The excised graft was fixed and dehydrated, and a paraffin section was then prepared and subjected to HE staining and immunohistological staining for the expression of the proteins of interest (insulin, glucagon, Ki67, human nucleus, PDX1, and CK19). The presence or absence of non-endocrine cells was confirmed on the basis of the morphology and staining image thereof. The number and frequency of non-endocrine cells appearing in the graft were counted as total numbers from four tests conducted with cells derived from a plurality of batches treated in the same manner.

### 2. Results

### (1) In vivo of insulin-producing cell population prepared by addition of docetaxel

Figure 6 shows results about a human C-peptide concentration in plasma and a graft weight measured in follow-up after transplantation. A subcutaneously transplanted insulin-producing cell population prepared by the addition of docetaxel exhibited human C-peptide secretion in plasma at the same level as in an insulin-producing cell population prepared without the addition of docetaxel, and this secretion was continuously confirmed up to 6 months after transplantation. A low tendency of the graft weight at 6 months was observed in the insulin-producing cell population prepared by the addition of docetaxel.

### (2) Evaluation of non-endocrine cells in graft and protein expression

In results of immunohistologically staining the graft 6 months after transplantation, many insulin-positive and glucagon-positive cells were confirmed throughout the whole graft of the insulin-producing cell population prepared by the addition of docetaxel, whereas Ki67-positive cells were slightly scattered. By contrast, the presence of insulin-positive and glucagon-positive cells as well as the appearance of many Ki67-positive cells was confirmed in the graft of the insulin-producing cell population prepared without the addition of docetaxel.

Table 9 shows the counted number and frequency of a non-endocrine cell population appearing as to results of four transplantation tests conducted using the insulin-producing cell population prepared by the addition of docetaxel. The appearance rates (percentages of the numbers of appearance) of Outgrowth and Cyst were 73% (16/22) and 100% (22/22), respectively, in a transplantation group of the insulin-producing cell population prepared without the addition of docetaxel (Docetaxel(-)), whereas the appearance rates were 0% (0/21) and 10% (2/21), respectively, in a transplantation group of the insulin-producing cell population prepared by the addition of docetaxel (Docetaxel(+)), confirming evident decrease in the appearance rate of non-endocrine cells in the graft.

This suggested that the addition of docetaxel in a culture step is effective for suppressing the appearance of unintended non-endocrine cells *in vivo* after transplantation. Outgrowth shown in Table 9 refers to a population of human nucleus-positive, PDX1-negative, and Ki67-positive non-endocrine cells, and its presence was determined from morphology by HE staining as well as by immunohistological staining. Cyst shown in Table 9 refers to human nucleus-positive, PDX1-positive, and CK19-positive non-endocrine duct cells, and its presence was determined from a duct form by HE staining as well as by immunohistological staining.

**[Table 9]**

| Cell type | The number of tests | Total number (the number of animals) | The number of appearance | | Frequency of appearance (%) | |
|---|---|---|---|---|---|---|
| | | | Outgrowth | Cyst | Outgrowth | Cyst |
| Docetaxel (-) | 4 | 22 | 16 | 22 | 73 | 100 |
| Docetaxel (+) | 4 | 21 | 0 | 2 | 0 | 10 |

In conclusion, the insulin-producing cell population prepared by the addition of docetaxel was found to suppress the proliferation of non-endocrine cells *in vivo* in a recipient and to be able to produce endocrine cells and insulin-producing cells at high proportions, and its efficacy and safety were confirmed.

## Claims

1. A method for producing an insulin-producing cell population, comprising
culturing a pancreatic endocrine progenitor cell population and/or a cell population at a later stage of differentiation to be treated in a medium containing a compound having tubulin polymerization-promoting activity and/or tubulin depolymerization-inhibiting activity.

2. The method according to claim 1, wherein the compound having tubulin polymerization-promoting activity and/or tubulin depolymerization-inhibiting activity is a compound selected from taxoid anticancer agents or a pharmacologically acceptable salt thereof.

3. The method according to claim 1 or 2, wherein the compound having tubulin polymerization-promoting activity and/or tubulin depolymerization-inhibiting activity is docetaxel or a pharmacologically acceptable salt thereof.

4. The method according to any one of claims 1 to 3, wherein the produced insulin-producing cell population comprises 95% or more of CHGA-positive cells.

5. The method according to any one of claims 1 to 3, wherein the produced insulin-producing cell population comprises 0.1% or less of CHGA-negative and Ki67-positive cells.

6. The method according to any one of claims 1 to 5, wherein the pancreatic endocrine progenitor cell population and/or the cell population at a later stage of differentiation to be treated is a cell population produced by the induction of differentiation from pluripotent stem cells.

7. A culture medium for a pancreatic endocrine progenitor cell population and/or a cell population at a later stage of differentiation, comprising a compound having tubulin polymerization-promoting activity and/or tubulin depolymerization-inhibiting activity.

8. The culture medium according to claim 7, wherein the compound having tubulin polymerization-promoting activity and/or tubulin depolymerization-inhibiting activity is a compound selected from taxoid anticancer agents or a pharmacologically acceptable salt thereof.

9. The culture medium according to claim 7 or 8, wherein the compound having tubulin polymerization-promoting activity and/or tubulin depolymerization-inhibiting activity is docetaxel or a pharmacologically acceptable salt thereof.

10. The culture medium according to any one of claims 7 to 9, wherein the culture medium is used for increasing the proportion of CHGA-positive cells and/or for increasing the proportion of insulin-positive and NKX6.1-positive cells.

11. A method for detecting non-endocrine cells in an insulin-producing cell population, comprising culturing the insulin-producing cell population in a medium containing epidermal growth factor (EGF).

12. The method according to claim 11, wherein the non-endocrine cells are CHGA-negative and PDX1-positive cells and/or CHGA-negative and PDX1-negative cells.

13. A method for removing non-endocrine cells, comprising
culturing a pancreatic endocrine progenitor cell population and/or a cell population at a later stage of differentiation to be treated in a medium containing a compound having tubulin polymerization-promoting activity and/or tubulin depolymerization-inhibiting activity.

14. A culture medium for an insulin-producing cell population, comprising EGF, wherein the culture medium is used for detecting non-endocrine cells in the insulin-producing cell population.
